# EUROPEAN PATENT SPECIFICATION

(11) **EP 2 999 795 B1**
(45) Date of publication and mention of the grant of the patent: **24.07.2019**
(21) Application number: 14725179.7
(22) Date of filing: 20.05.2014
(51) Int. Cl.: C12Q 1/68, C12N 15/10, C12N 15/63, C12N 15/67, C40B 40/02, C40B 40/06

(54) **REGULATABLE GENE EXPRESSION**
REGULIERBARE GENEXPRESSION
EXPRESSION RÉGLABLE DES GÈNES

(30) Priority: 21.05.2013 GB 201309132
(43) Date of publication of application: 30.03.2016
(73) Proprietor: Biosyntia ApS, 2100 Copenhagen (DK)
(72) Inventor: GENEE, Hans Jasper, 2200 København N (DK); SOMMER, Morten, 2830 Virum (DK)
(74) Representative: Birkeland, Morten
(86) International application number: PCT/EP2014/060353
(87) International publication number: WO 2014/187829

(56) References cited:
- EP-A1- 1 801 212
- WO-A2-2012/153142
- US-A1- 2012 244 601
- DESAI SHAWN K ET AL: "Genetic screens and selections for small molecules based on a synthetic riboswitch that activates protein translation", JOURNAL OF THE AMERICAN CHEMICAL SOCIETY, ACS PUBLICATIONS, US, vol. 126, no. 41, 20 October 2004 (2004-10-20), pages 13247-13254, XP002567682, ISSN: 0002-7863, DOI: 10.1021/JA048634J [retrieved on 2004-09-24] cited in the application
- JINA YANG ET AL: "Synthetic RNA devices to expedite the evolution of metabolite-producing microbes", NATURE COMMUNICATIONS, vol. 4, 29 January 2013 (2013-01-29), page 1413, XP055130450, DOI: 10.1038/ncomms2404
- NICHOLAS J TURNER: "Directed evolution drives the next generation of biocatalysts", NATURE CHEMICAL BIOLOGY, vol. 5, no. 8, 1 August 2009 (2009-08-01), pages 567-573, XP055130457, ISSN: 1552-4450, DOI: 10.1038/nchembio.203
- N. MURANAKA ET AL: "An efficient platform for genetic selection and screening of gene switches in Escherichia coli", NUCLEIC ACIDS RESEARCH, vol. 37, no. 5, 1 January 2009 (2009-01-01), pages e39-e39, XP055130803, ISSN: 0305-1048, DOI: 10.1093/nar/gkp039
- SINHA J ET AL: "From SELEX to cell: Dual selections for synthetic riboswitches", METHODS IN ENZYMOLOGY, ACADEMIC PRESS, US, vol. 497, 1 January 2011 (2011-01-01), pages 207-220, XP009179267, ISSN: 0076-6879, DOI: 10.1016/B978-0-12-385075-100009-3
- YOKO NOMURA ET AL: "Synthetic mammalian riboswitches based on guanine aptazyme", CHEMICAL COMMUNICATIONS, vol. 48, no. 57, 1 January 2012 (2012-01-01), page 7215, XP055131142, ISSN: 1359-7345, DOI: 10.1039/c2cc33140c
- Lee Ronald Ferguson: "Regulation of gene expression for therapy of age related macular degeneration", , 1 January 2009 (2009-01-01), XP055131047, ISBN: 978-1-12-413349-2 Retrieved from the Internet: URL:http://search.proquest.com/docview/744 519526

### SEQUENCE LISTING

<110> Biosyntia ApS
<120> Regulatable Gene Expression
<160> 21
<170> BiSSAP 1.2
<210> 1
   <211> 48
   <212> DNA
   <213> Artificial Sequence
<220>
   <221> source
   <222> 1..48
   <223> /mol_type="unassigned DNA" /note="GEBO1" /organism="Artificial Sequence"
<400> 1
   attctctaga aaguatagga acttctctaa gaaaccatta ttatcatg 48
<210> 2
   <211> 43
   <212> DNA
   <213> Artificial Sequence
<220>
   <221> source
   <222> 1..43
   <223> /mol_type="unassigned DNA" /note="GEBO2" /organism="Artificial Sequence"
<400> 2
   actttctaga gaauaggaac ttccgaaagg gcctcgtgat acg 43
<210> 3
   <211> 33
   <212> DNA
   <213> Artificial Sequence
<220>
   <221> source
   <222> 1..33
   <223> /mol_type="unassigned DNA" /note="GEBO3" /organism="Artificial Sequence"
<400> 3
   acttcggguc ctcaacgaca ggagcacgat cat 33
<210> 4
   <211> 33
   <212> DNA
   <213> Artificial Sequence
<220>
   <221> source
   <222> 1..33
   <223> /mol_type="unassigned DNA" /note="GEBO4" /organism="Artificial Sequence"
<400> 4
   acccgaagut cctattctct agaaagtata gga 33
<210> 5
   <211> 33
   <212> DNA
   <213> Artificial Sequence
<220>
   <221> source
   <222> 1..33
   <223> /mol_type="unassigned DNA" /note="GEBO5" /organism="Artificial Sequence"
<400> 5
   agtgatctuc ttctccatct tgttgttacc tta 33
<210> 6
   <211> 27
   <212> DNA
   <213> Artificial Sequence
<220>
   <221> source
   <222> 1..27
   <223> /mol_type="unassigned DNA" /note="GEBO6" /organism="Artificial Sequence"
<400> 6
   aagatcacug gatataccac cgttgat 27
<210> 7
   <211> 22
   <212> DNA
   <213> Artificial Sequence
<220>
   <221> source
   <222> 1..22
   <223> /mol_type="unassigned DNA" /note="GEBO29" /organism="Artificial Sequence"
<400> 7
   ggcgtatcac gaggcccttt cg 22
<210> 8
   <211> 20
   <212> DNA
   <213> Artificial Sequence
<220>
   <221> source
   <222> 1..20
   <223> /mol_type="unassigned DNA" /note="GEBO31" /organism="Artificial Sequence"
<400> 8
   ccgggctgca ggaattcgat 20
<210> 9
   <211> 28
   <212> DNA
   <213> Artificial Sequence
<220>
   <221> source
   <222> 1..28
   <223> /mol_type="unassigned DNA" /note="GEBO42" /organism="Artificial Sequence"
<400> 9
   aagtttacuc gaacttccat atgaggcc 28
<210> 10
   <211> 31
   <212> DNA
   <213> Artificial Sequence
<220>
   <221> source
   <222> 1..31
   <223> /mol_type="unassigned DNA" /note="GEBO43" /organism="Artificial Sequence"
<400> 10
   actccgcuag cgcgccgaac gaccgagcgc a 31
<210> 11
   <211> 30
   <212> DNA
   <213> Artificial Sequence
<220>
   <221> source
   <222> 1..30
   <223> /mol_type="unassigned DNA" /note="GEBO44" /organism="Artificial Sequence"
<400> 11
   agcggagugt atactggctt actatgttgg 30
<210> 12
   <211> 28
   <212> DNA
   <213> Artificial Sequence
<220>
   <221> source
   <222> 1..28
   <223> /mol_type="unassigned DNA" /note="GEB045" /organism="Artificial Sequence"
<400> 12
   atggacaacu tatatcgtat ggggctga 28
<210> 13
   <211> 31
   <212> DNA
   <213> Artificial Sequence
<220>
   <221> source
   <222> 1..31
   <223> /mol_type="unassigned DNA" /note="GEB046" /organism="Artificial Sequence"
<400> 13
   agttgtccau atgaatatcc tccttaggaa t 31
<210> 14
   <211> 28
   <212> DNA
   <213> Artificial Sequence
<220>
   <221> source
   <222> 1..28
   <223> /mol_type="unassigned DNA" /note="GEB047" /organism="Artificial Sequence"
<400> 14
   agtaaactug gtctgacagt taccaatg 28
<210> 15
   <211> 25
   <212> DNA
   <213> Artificial Sequence
<220>
   <221> source
   <222> 1..25
   <223> /mol_type="unassigned DNA" /note="GEB072" /organism="Artificial Sequence"
<400> 15
   acaagaugag ggaagcggtg atcgc 25
<210> 16
   <211> 27
   <212> DNA
   <213> Artificial Sequence
<220>
   <221> source
   <222> 1..27
   <223> /mol_type="unassigned DNA" /note="GEB073" /organism="Artificial Sequence"
<400> 16
   atcttgutgt taccttagca gggtgct 27
<210> 17
   <211> 29
   <212> DNA
   <213> Artificial Sequence
<220>
   <221> source
   <222> 1..29
   <223> /mol_type="unassigned DNA" /note="GEB074" /organism="Artificial Sequence"
<400> 17
   atcctgacgg utgacggcta gctcagtcc 29
<210> 18
   <211> 29
   <212> DNA
   <213> Artificial Sequence
<220>
   <221> source
   <222> 1..29
   <223> /mol_type="unassigned DNA" /note="GEB077" /organism="Artificial Sequence"
<400> 18
   accgtcagga ucaacgttca aatccgctc 29
<210> 19
   <211> 34
   <212> DNA
   <213> Artificial Sequence
<220>
   <221> source
   <222> 1..34
   <223> /mol_type="unassigned DNA" /note="Flippase Recognition Target " /organism="Artificial Sequence"
<400> 19
   gaagttccta ttctctagaa agtataggaa cttc 34
<210> 20
   <211> 1317
   <212> DNA
   <213> Artificial Sequence
<220>
   <221> source
   <222> 1..1317
   <223> /mol_type="unassigned DNA" /note="Xanthine Permease 1H AB95D01" /organism="Unknown"
<400> 20
   atgaatcatc ggcaaaagaa tgcgatttct ccagagcatg aatatttagg aatatccaaa      60
   agttttgctt acgggttaca gcacgtgctt accatgtacg gtggcattgt tgcaccgccc     120
   cttattattg gaacagcggc tggattaagc tcagcacaag tcggaatgct aatcgctgct     180
   gctctatttg tcggtggatt ggcaacactg attcaaacaa ttgggaccaa atatctaggt     240
   gcaaaattac ctttagttca aggcgtgtct tttgcaggtg ttgcaactat ggtcgcaatt     300
   atcaccacag gcggtggctt acctgctgta tacggtgcgg tcattgttgc gtctctgatt     360
   ggcctatgtc ttgcacctta tttttctaaa attattcgat tttttccacc tgttgtaacg     420
   ggatgcgtca tcacaattat tggtttatct ctgttacctg tcgcgattcg ctggatgatg     480
   ggtggtaaca ataaagcacc cgactggggt agtgtagaaa atatctcttt ggccttgctg     540
   accttaggca ttgtgattgt tttaaatatg ctgccgcaag caagcattcg tcgcttatcg     600
   attttattgg ctattgttgc tggtactgcc ctcgcctact tcatgggttt tggtgatttt     660
   agtcaggtga gctcaggcgc atggctgcaa tttccacact tatttgcatt tggcttaccg     720
   actttcgagt taagcgcaat cttgtcgatg ttgattgtga ctttagtcat tatgacggaa     780
   accacagccg atattattgc tgtcggagac attgtaggaa ctgaagtaga tgctaaacgt     840
   atcgccaatg gtgtacgtgc ggatatgttt tcaagtgctt ttgcgcctat ctttggttcg     900
   tttatgcaaa gtgcttttgc ccaaaatgtt ggactcgttg cgattacagg cataaaaagc     960
   cgcttcgtag tagcagcagg cggtgtcatt ctgattattt tgggtctact ccctattatg    1020
   ggtcgtttga ttgcagcgat tccaatgccc gtacttggtg gtgcaggttt ggtcttattt    1080
   ggatcggttg cagcaagtgg aattcgcact ttggcaaaaa tagattataa cgatcagaaa    1140
   aatttaatta ttgtcgcgac cgctttatcg gcaggtatga ttccaattat taaccatgaa    1200
   ttttatgctc atttcccagt ttgggtacaa actttgttcc attcgggcat tagttcaact    1260
   tgtatctttg ctatcttgtt gaatttgtta tttaatcatt tacgacggta tcgataa       1317
<210> 21
   <211> 1416
   <212> DNA
   <213> Artificial Sequence
<220>
   <221> source
   <222> 1..1416
   <223> /mol_type="unassigned DNA" /note="Xanthine Permease 4G 57SDb01" /organism="Unknown"
<400> 21
   atggagaaaa ggaacgattt ctcagatttt ctttgtaatt ttgccgccaa attcagaaaa      60
   gacactgaat tgatggataa tggaaattct ctaattatta taacgacaat ggaacagtca     120
   atagaaaatc tctacaaatt agacggcaga gtgcccgttg gtaaagcact cccatttggc     180
   cttcagcatg tgctggcgat gttcgtcagc aatattgcgc caatcatgat tctcgctggt     240
   gcgataggtt tggacagttc ggtgagcgcc gtgcttatcc agaactgtat ggtcatcgcc     300
   ggtatcggta ccttggtgca gctttatccg gtatggcgta tcggttctcg tcttcctatc     360
   gtgatgggta tctccttcac cttcctttcg ctcgccatcg ccattgccgg tgctcatggt     420
   atgggtacat tgataggcgc tgtcatcatt ggtggtctgg tagaaggaac actcggactt     480
   ttcgccaagt attggatcaa gctcattccg cctgtagtag ccgctaccgt ggtaacagcc     540
   atcggtttct cgctccttcc tgttggtgcc aactcttttg cgggtggtca gggtgcagct     600
   gatttcggta gcatgcacaa ctgggttgtg ggttctgtca ctttgctggc ttgtctgctt     660
   tgtcagatct tcgccaaggg tttcttgcgt tctctctctg tattggtagg actgctggta     720
   ggttatgttt tggcactgtt tatgggaatg atagatttca gtggactttc tggtctctct     780
   atcgtagctt tgccaaagct tcttccattt acacctgagt ttaatatcgg tgctattctc     840
   tcggtggtag cagtatatct ggtttctgcc acagagacga ttggtgatac ttctgcgctc     900
   tgcaacagtg cgttgaagcg tgatcctaag accaaggaga tgggtgcggc agtctgctgc     960
   gatggtttcg tcagttcggt ttccggtctt ttcggttgta ctcctatcac ttctttcagt    1020
   cagaacgtag gtttggctgc gatgtcggga gtggtgaacc gctttactat tgccatgggt    1080
   gccatcatca tgattatcgg tggtatcttc ccagctatcg gttatgtatt gactacgatt    1140
   cctcaggctg tattgggcgg ctgtaccatc atgatgttcg gtagcatcct ctttgccggt    1200
   ttcggcatga tggcgcgtac cggcttctct cagcgcaaca tggtcatcgt gagcctctcg    1260
   ctcagcgtag gtctcggatt cacatctgca acaggtatgt tcaacatctt cccagagatt    1320
   gtgcgcaccg tttttgctga taactgtgtg gcagtagtct tcctgcttgc cgttatcctg    1380
   aacctcgtgt tgccgaagaa tctggataag gcataa                              1416

## Description

### Technical Field

The present invention relates to a method of employing a regulatable gene expression construct in an in vivo selection procedure.

### Background of the Invention

Biocatalysts are biologically produced catalysts which can activate or accelerate the rate of chemical reactions. Biocatalysis is becoming an increasingly important tool in the chemical industry, particularly in the paper, leather, personal care and detergent industries (Sanchez & Demain, 2011). Biocatalysts can comprise whole cell systems, as well as isolated enzymes; the latter are generally categorised as industrial and bioconversion enzymes.

Biocatalytic enzymes are playing an ever more crucial role in organic synthesis processes. An increasing number of industrial products, ranging from pharmaceutical intermediates to bulk chemicals, are manufactured with processes that involve one or more biocatalytic steps, foretelling the emergence of a significant biotechnology based chemical industry.

The continued success of the biocatalyst-based chemical industry is reliant on increasing the availability of biocatalysts that enable good overall process efficiency and low production costs. This requires the continuous development of cell systems and/or enzymes that show the necessary activity and stability for desired bioconversions under practical process conditions.

Much effort is devoted to finding or developing enzymes that meet these criteria, both in academia, where research centers focusing on biocatalysis have recently become popular, and in industry, where growing numbers of young biotech and established pharmaceutical and chemical companies are now developing new biocatalysts.

In principle, the biocatalytic diversity available in the biosphere is immense. This natural diversity can be synthetically extended in the laboratory using, for example, DNA shuffling, directed evolution or site directed mutagenesis techniques. Strategies for enzyme discovery and optimization are generally based on screening these resources. All applied biocatalysts, including industrial enzymes as well as biocatalysts for large scale production of chemicals and pharmaceuticals, have been discovered by extensive and persistent screening of a large number of microorganisms from nature (Lorenz & Eck, 2004). However, finding desired biocatalysts in this vast pool currently utilises slow and costly screening techniques which limits the development of new biocatalysts.

There is an evident need for improved methods for producing biocatalysts. Rational redesign for the creation of new optimised biocatalysts is exceptionally challenging. A few examples do exist (Nestl, Nebel & Hauer, 2011), but the contribution is still minor. Even re-designing existing enzymes to produce new optimised biocatalysts has been performed with only limited success (N. J. Turner, 2009). However, such approaches are still in their infancy and may yet prove to be more fruitful in due course.

The initial phase of enzyme discovery typically involves screening an available culture of microorganisms in the laboratory. However, it is estimated that less than 1% of all microorganisms in nature can be cultivated by current laboratory procedures, probably due to unmet complex nutritional requirements or symbiotic interdependencies (Kaeberlein, Lewis & Epstein, 2002).

Techniques that allow isolation, cloning and recombinant expression of environmental DNA have made activity based screening of enzymes from non-cultivable organisms possible (Lorenz et al, 2003). The overall procedure follows four primary steps:
1. Extraction of DNA from a natural substratum
2. Fragmentation of DNA to sizes that depend on the purpose of the library. For expression based screening 1-120 kb is typically used and cloned into vectors such as plasmids, fosmids, cosmids, phages and BACs
3. For functional screening/selection strategies the gene library is cloned into a surrogate host to construct a metagenomic expression library.
4. The library of cells with unique inserts is evaluated by screening/selection.

This procedure generates a metagenomic library of from 10⁶ - 10¹⁰ variants, which vastly increases the likelihood of the presence of a microorganism which produces a desired biocatalyst. The problem which remains to be solved, however, is how to successfully and easily screen such a vast library of variants for a desired biocatalyst.

### Library evaluation for enzyme identification

For activity based screening, there are generally two options for library evaluation: high-throughput screening (HTS) and in vivo selection.

Aharoni et al (Curr. Op. Chem. Biol., 2005) discloses a HTS method of visualising enzymatic activity through the use of tailored assays involving the conversion of fluorogenic or chromogenic substrates to spectroscopically different products by the enzyme of interest.

Leemhuis et al (IUBMB life, 2009) reviews various HTS techniques, including FACS, cells in droplets, cell surface display, and in vitro compartmentalisation with ultra high-throughput capacities. Such techniques have been found to produce high levels of false positive results (Dietrich, McKee & Keasling, 2010).

A significant disadvantage of using HTS methods is that such screening methods often require each individual of the library to be tested separately, which means that the methods are frequently dependent on costly automation.

Sommer, Dantas & Church (Science 2009, Mol. Sys. Bio. 2010) disclose in vivo selection methods of screening metagenomes for resistance/detoxifying genes, such as antibiotic resistance genes, and genes that increase tolerance to growth inhibitors. Steele, Jaeger, Daniel & Streit (J. Mol. Microbiol. Biotechnol., 2009) review in vivo selection methods.

Generally, in vivo selection methods require that the expressed enzyme confer a significant biological advantage to the host cell, such as allowing the host cell to grow in growth inhibiting conditions.

EP 1801212 (ETHZ, 2005) discloses an in vivo method of selecting from a population of candidate biocatalysts a biocatalyst capable of catalysing a chemical reaction from a substrate to a product by providing a host cell comprising a product-inducible expression system and a biocatalyst expression system and growing said host cell in growth inhibiting conditions. Cells which contain a desired biocatalyst effect a chemical transformation of a substrate to a product, which said product binds to the product-inducible expression system, the expression of which allows proliferation in the growth inhibiting conditions and therefore selection of said biocatalyst. This system is effective for smaller populations of cells (∼10⁵ cells) but is less effective for larger populations, with 10-30% false positives observed with libraries comprising ∼10⁶ cells.

Yang et al. (Nature Communications, 2012) discloses a method of using a synthetic riboswitch which binds lysine to select from among a population of host cells those cells which produce high levels of lysine from those that do not produce high levels of lysine.

US 2013/0310458 discloses a genetically modified cell which comprises a gene sequence coding for an autofluorescent protein which is operatively linked to a riboswitch, wherein the expression of the autofluorescent protein depends on the intracellular concentration of an aptamer binding metabolite.

WO2012/153142A2 discloses a genetic system to regulate gene expression using riboswitches as a regulatory tool; wherein one aspect provides a construct comprising a first riboswitch operably linked to a coding sequence and a second riboswitch operably linked to a second coding sequence to allow for variable co-expression of the different coding sequences.

Gallivan (J. A. Chem. Soc., 2004) provides a method of using a synthetic riboswitch that, in the presence of theophylline, activate translation of a coding region encoding antibiotic resistance, allowing proliferation of a cell harboring said riboswitch. Selection of a cell harboring said synthetic riboswitch with particular ligand specificity for theophylline from a million-fold larger pool of cells containing mutant riboswitches was demonstrated. A similar method is disclosed in US 2012/0244601 (Gallivan, 2012).

It could be envisaged that the selection procedures in the above cited art could be further developed to detect transporter compounds - compounds which transport substrates from the extracellular space into the intracellular space. The product-inducible selection systems could be used to select compounds which transport said product (which is inert to passive cellular diffusion) from the extracellular space into the cell. Substrates which are inert to passive diffusion from the extracellular space to the intracellular space can be problematic when engineering in vivo biochemical syntheses which require said substrates, therefore providing a method for selecting genes which encode transporter compounds for transporting a specific substrate ('product') across the cell membrane would prove beneficial for cellular engineering.

Developmental work towards the current invention found the method as disclosed in Gallivan produced significant levels of false positive results, potentially due to the riboswitch being 'leaky' (the coding region is expressed even in the absence of theophylline).

Thus, the aim of the present invention is to provide a general system which uses a riboswitch regulation mechanism for an in vivo selection of biocatalysts and/or transporter compounds (and genes thereof), wherein said system is fast, cost effective and can be used in a high throughput mode whilst firmly maintaining a negligible level of false positive results.

### Summary of the Invention

The present invention now relates to an improved in vivo selection procedure for biocatalysts and/or transporter compounds, herein collectively referred to as a 'primary modulator compound', through the use of a regulatable gene expression construct controlled by the presence in a host cell of an effector compound, the presence of which is dependent on the presence of said primary modulator compound, wherein said primary modulator compound is a functional molecule capable of modulating the action of said effector compound. The use of a multiple regulatory selection system comprising two or more riboswitches which bind the same effector compound, described herein, reduces the level of false positive results to negligible levels whilst maintaining high selectivity for host cells comprising said regulatory selection system and said primary modulator compound.

In one aspect the present invention relates to a method for the in-vivo selection of a genetic coding sequence encoding at least one primary modulator compound modulating the action of an effector compound, comprising growing an expression library of host micro-organisms in the presence of at least two growth regulator compounds each capable normally of preventing proliferation of said micro-organisms, which expression library expresses a library of DNA fragments potentially expressing said primary modulator compound , and selecting micro-organisms in said library which proliferate; wherein said primary modulator compound
- effects a chemical transformation of a substrate into said effector compound and said substrate is provided to said microorganisms; or
- transports said effector compound into said micro-organisms, and
wherein said host microorganisms comprise a regulatable gene expression construct, said regulatable gene expression construct comprising a nucleic acid molecule, said nucleic acid molecule comprising at least two regulation sequences, each said regulation sequence encoding an RNA molecule comprising a riboswitch operably linked to a respective coding region, wherein each riboswitch regulates expression of its respective coding region in response to the presence of said effector compound, and each coding region encodes a respective secondary modulator compound for modulating the action of a respective said growth regulator compound, and wherein said at least two regulation sequences encode different secondary modulator compounds that act against different said at least two growth regulator compounds; whereby a micro-organism in said library which produces a desired primary modulator compound is enabled to proliferate in the presence of said at least two growth regulator compounds.

In a preferred embodiment, said expression library of host micro-organisms comprises a multitude of host cells that produces a population of potential primary modulator compounds, wherein said multitude of host cells:
- is a library of cells of a single cell type wherein essentially each host cell comprises a cloned nucleic acid fragment or mutated native genome encoding at least one potential primary modulator compound; or
- are cells of different cell types wherein essentially each host cell comprises a native nucleic acid fragment encoding at least one potential primary modulator compound.

In another preferred embodiment, said host micro-organism is contacted with said substrate in a growth medium comprising at least two antibiotic compounds as said growth regulator compounds for which resistance for each antibiotic compound is encoded in each of the operably linked coding regions, such that each operably linked coding region encodes resistance for one antibiotic compound. Preferably, said coding regions encode respective enzymes conferring antibiotic resistance.

In another preferred embodiment, at least one micro-organism host cell comprises one or more nucleic acid molecules encoding at least two primary modulator compounds each capable of catalysing at least one reaction of a multi-step chemical conversion reaction, and wherein at least one of said chemical conversion reactions produces said effector compound.

In yet another preferred embodiment, said micro-organism host cell comprises one or more nucleic acid molecules encoding at least two potential primary modulator compounds, wherein at least one primary modulator compound effects a chemical transformation of a substrate into said effector compound and at least one primary modulator compound transports said substrate into said microorganism.

In yet another preferred embodiment, said method for the in-vivo selection further comprises selecting the genetic coding sequence encoding at least one primary modulator compound from micro-organisms in said library which proliferate; and introducing the selected genetic coding sequence into a micro-organism host cell by transgenic methods thereby producing a production strain, wherein said production strain produces said primary modulator compound or compounds.

In another aspect the present invention relates to a library of micro-organism host cells suitable for use in a method of selecting among a population of potential primary modulator compounds a primary modulator compound, each said host cell comprising;
a) at least one nucleic acid molecule encoding at least one potential primary modulator compound,
   wherein said primary modulator compound
   - effects a chemical transformation of a substrate into said effector compound and said substrate is provided to said microorganism host cells; or
   - transports said effector compound into said micro-organism host cells; and
b) at least one regulatable gene expression construct comprising a nucleic acid molecule encoding at least two or more regulation sequences, each said regulation sequence encoding an RNA molecule comprising a riboswitch operably linked to a respective coding region which encodes a respective secondary modulator compound for modulating the action of a respective growth regulator compound, wherein said at least two regulation sequences encode different secondary modulator compounds that act against different said at least two growth regulator compounds; and wherein each riboswitch regulates expression of its respective coding region in response to the presence of an effector compound.

In another aspect the present invention relates to a micro-organism host cell, wherein said host cell comprises
(a) at least one regulatable gene expression construct wherein said regulatable gene expression construct comprises a nucleic acid molecule; wherein said nucleic acid molecule comprises two or more regulation sequences, each said regulation sequence encoding an RNA molecule comprising a riboswitch responsive to an effector compound, each said riboswitch being operably linked to a respective coding region; wherein each coding region encodes a respective modulator compound for modulating the action of a respective growth regulator compound; wherein said two or more regulation sequences encode different secondary modulator compounds that each act against a different growth regulator compound; and wherein the riboswitch in each regulation sequence is selected to be responsive to the same effector compound to trigger expression of its respective modulator compound; and
(b) at least one nucleic acid molecule encoding at least one primary modulator compound, wherein said primary modulator compound
   - effects a chemical transformation of a substrate into said effector compound; or
   - transports a compound from the extracellular space to the intracellular space, wherein said transporter compound is said substrate or is said effector compound.

In a final aspect the present invention relates to a method of producing a primary modulator compound production strain, wherein the selected genetic coding sequence or sequences selected according to the herein described method is introduced into a micro-organism host cell by transgenic methods thereby producing a production strain, wherein said production strain produces said primary modulator compound or compounds.

### Definitions

"Effector compound" as used herein refers to a compound which binds to a riboswitch and causes a conformational change in said riboswitch thereby regulating the expression of its respective coding region.
"Primary modulator compound" as used herein refers to a compound which modulates the action of an effector compound, wherein said primary modulator compound either effects a chemical transformation of a substrate into an effector compound or transports said effector compound into a micro-organism.
"Secondary modulator compound" as used herein refers to a compound which modulates the action of a growth regulator compound.
"Growth regulator compound" as used herein refers to a compound which suppresses cellular growth such that a cell which is in the presence of said growth regulator compound no longer proliferates.
"Cloning" is referred to as the process of inserting DNA into a vector, then establishing it as a stable part of a cell line.
The term "expression vector" as used herein refers to a DNA molecule that is used to introduce and express a specific nucleic acid sequence into a target cell. Once the expression vector is inside the cell, the potential primary modulator compound that is encoded by the nucleic acid sequence is produced by the cellular transcription and translation machinery. Generally, these expression vectors include regulatory elements operably linked to the nucleic acid of the encoding specific gene (i.e. the nucleic acid for the candidate biocatalyst and/or the detector gene). "Transcription" refers to the synthesis of RNA on a DNA or RNA template. "Translation" refers to the synthesis of protein on the mRNA template. Nucleic acid is "operably linked" when it is placed into a functional relationship with another nucleic acid sequence. For example, a riboswitch is operably linked to a coding sequence if it affects the translation of said sequence.

### Figures

The invention will be further described and illustrated with reference to the accompanying drawings in which:-
Figure 1 shows the 221 base pair DNA sequence (from terminator to start codon) which constitutes the 'Riboswitch Platform', expression of which said sequence produces a riboswitch specific for binding theophylline.
Figure 2 shows the pGEB05.1 vector carrying the dual riboswitch selection system.
Figure 3 illustrates a cell comprising the production routes of the two types of primary modulator compound which modulate the action of the effector compound.
Figure 4 illustrates a preferred embodiment of the positive selection method.
Figure 5 illustrates a preferred embodiment of the negative selection method.

### Outline of a preferred embodiment

The following provides further detail on the workings of the selection method as described herein.

### Basic system layout

The general approach is to detect the presence of a host cell comprising a primary modulator compound which modulates the action of an effector compound by using two or more riboswitches which both recognise said effector compound. Each riboswitch controls the expression of a coding region which in turn confers a survival capability upon the cell. The use of at least two riboswitches is crucial for this method to succeed, as this significantly reduces the chance of spontaneous resistance developing in the host cell.

By coupling detection of an effector compound to survival under selective conditions, the system links growth to intracellular formation of a primary modulator compound.

### Riboswitch

Riboswitches are the expression control elements of the RNA molecule to be expressed and that change conformational state when bound by an effector compound. Riboswitches typically can be dissected into two separate domains: one that selectively binds the target (aptamer domain) and another that influences genetic control (expression platform domain). It is the dynamic interplay between these two domains that results in control of gene expression.

Riboswitches function in two distinct modes: OFF and ON.

OFF riboswitches ('Negative Selection' as used herein) are riboswitches which shut down gene expression upon binding an effector compound to the aptamer domain (i.e. the conformational change conceals the ribosome binding site which disallows expression of the associated gene).

ON riboswitches ('Positive Selection' as used herein) are riboswitches which allow gene expression upon binding an effector compound to the aptamer domain (i.e. the conformational change exposes the ribosome binding site which allows expression of the associated gene).

'Switching' of a riboswitch herein refers to the change in state of the riboswitch upon binding of an effector compound to the riboswitch. An effector compound is a molecule(s) and/or compound(s) that can effect the switching of a riboswitch. This includes the natural or normal effector compound which causes switching of the riboswitch and other compounds that can cause switching of the riboswitch. Riboswitches to be used in the current invention generally can be from any source, including naturally occurring riboswitches, chimeric riboswitches, engineered riboswitches, and recombinant riboswitches. Riboswitches are described in U.S. Patent Application Publication No. US 2005/0053951, U.S. Pat. No. 6,831,171, PCT Application Publication No. WO 2006/055351, U.S. Provisional Patent Application No. 60/625,864, U.S. Patent Application No. US 2009/0305253, European Patent Application No. EP 2322535, and PCT Application Publication No. WO 2011/088076, each of which is hereby incorporated for the description of riboswitches and their function.

The riboswitch is required to be selectable such that it possesses specific binding for the desired effector compound, such that the riboswitch will be responsive to said effector compound, so that said host cell will proliferate in its presence. Methods for screening and selecting riboswitches and/or compounds which activate riboswitches can be found in the art, for example U.S Patent Application No. US 2009/0305253 and US 2013/0004980, European Patent Application No. EP 2322535, and PCT Application Publication No. WO 2011/088076, each of which is hereby incorporated for the method of screening and selecting riboswitches.

### Coding region

As used herein, the "coding region" encodes a secondary modulator compound and relates to either:
- Positive selection: genes and/or nucleic acid sequences, the expression of which produces a compound or compounds which modulates the action of a growth regulator compound, optionally by chemically altering said growth regulator compound, such that the growth regulator compound no longer inhibits growth of the host organism comprising said coding sequence. Such chemical alteration may be, but is not limited to, chemical inactivation of a toxic or growth inhibiting compound or chemical modification of a carbon source such that said modified carbon source can be utilised by an auxotrophic microorganism.
- Negative selection: genes and/or nucleic acid sequences, the expression of which produces a compound or compounds which can chemically alter a non-cytotoxic compound such that said compound becomes cytotoxic (i.e. a growth regulator compound) which inhibits cell growth.

The coding regions in the current invention are operably linked to riboswitches. The purpose of the coding regions is to allow the selection of one or more host cells that contain a primary modulator compound on the basis that binding the effector compound to the aptamer of the riboswitch causes a change in the riboswitch such that the resultant change in the expression of its respective coding region allows cell growth.

Each independent coding region in the herein described regulatable gene expression construct encodes a different secondary modulator compound such that each secondary modulator compound acts against a different growth regulator compound.

A wide variety of genes known in the art are suitable for use as coding regions in the present invention. For positive selection such genes include, but are not limited to, genes for antibiotic resistance, genes that complement an auxotrophy in the host cell and other genes that allow growth under specific conditions that do not permit growth of host cells that do not contain or express these same genes. Such conditions include, but are not limited to, the inability of host cells to utilize various possible C-sources, the presence in the growth medium of toxic chemicals, protein toxins, or toxic metals. For negative selection such genes include, but are not limited to, URA3 (expression of which is lethal in the presence of 5-Fluoroorotic acid) and SacB (expression of which is lethal in the presence of sucrose).

### Biocatalyst

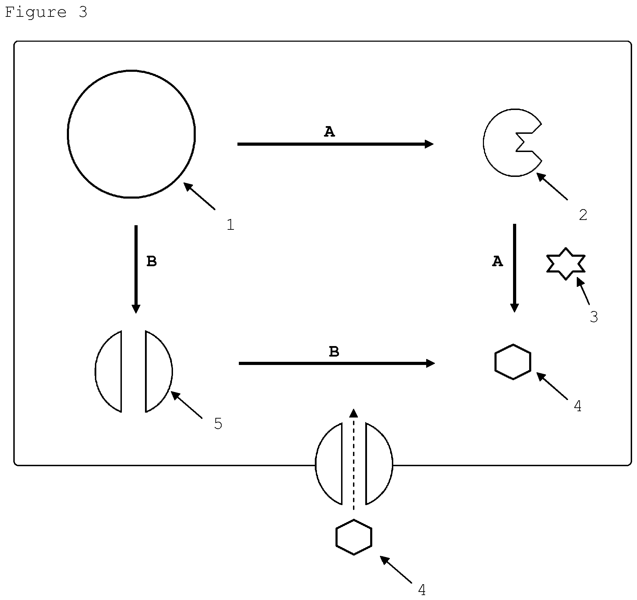

The term "biocatalyst" is used herein to refer to a primary modulator compound that effects a chemical transformation of a substrate into a product, wherein said product may be an effector compound. The production of a "biocatalyst" is illustrated in Figure 3, route **A:** the cloned DNA fragment, 1, is expressed producing the primary modulator compound, 2, which converts a substrate, 3, into an effector compound, 4.

In many instances, the biocatalyst will be an enzyme, however, it may also be a complex of enzymes, a combination of an enzyme and a co-factor, a biocatalytic RNA molecule, etc. The term is intended to cover all forms of the compound(s), both in the form of a protein, possibly modified by covalent or non-covalent attachment of sugars, oligosaccharides, polysaccharides or fatty acids, lipids, steroids and other chemical substituents and compounds, in the form of a nucleic acid or in the form of a combination of several such compounds. Generally, the true nature of the biocatalyst may first be established or assessed after its selection using the method of the present invention.

The population of potential biocatalysts from which a biocatalyst is to be selected in the method of the present invention is very suitably provided in the form of a library of nucleic acid molecules. Such nucleic acid molecules may comprise much more information than that encoding the biocatalyst itself. However, this is of no consequence to its possible selection. A "library" can be produced by digesting all DNA from a suitable source organism with a restriction enzyme or by sonication and cloning the fragments into a vector, which will result in the production of many different recombinant vectors, each with a different fragment of DNA cloned into it. The collection of many different recombinant vectors together forms the "library". The library may be produced by any method available to the skilled person, for instance by shotgun cloning. "Shotgun cloning" is referred to as using the whole genome of an organism as the starting point for cloning. Alternatively, DNA/RNA can be isolated from the environment, resulting in thousands of different genes which may have derived from many different organisms; such a collection of nucleic acid material is referred to as a metagenome. At the other end of the spectrum, specific libraries may be constructed that contain only genes expected to encode the desired biocatalysts, based for instance on DNA homologies, previous screening experiments, or commercially available libraries. A library constructed by shotgun cloning techniques may contain from a few thousand (microorganisms) to hundreds of thousands (higher eukaryotes or microbial metagenomes) of different recombinant plasmids while only one or a few comprise a sequence of interest. In order to reduce the size of libraries derived from eukaryotic organisms, it is sometimes advantageous to produce cDNA libraries. Since protein coding regions generally account for only a small percentage of the total genome size of eukaryotic organisms and since eukaryotic cells generally express only a subset of their genes, the production of cDNA copies of the cellular mRNA using reverse transcriptase and the cloning thereof into a cDNA library results in a library of reduced size comprising only the part of the organism's genome that is of most interest (the protein coding sequences that are expressed). cDNA library construction is well known to the skilled person. Other library types include, but are not limited to, a library obtained by i) mutation (directed or random) of a single parent gene ii) recombination of several parent genes (such as libraries produced by gene shuffling) iii) a combination thereof.

The "library" may be a library of genes or a library of organisms.

In the case the library is a library of organisms, the host may already have the desired biocatalytic activity, and the aim of the invention is to develop an improved biocatalyst. The population of potential biocatalysts may for instance be provided in the form of a mixed population of potentially biocatalytically active bacterial cells, i.e. cells of different strains, for instance isolated from a soil or water sample, to which cells the regulatable gene expression construct is added, in order to select the best strain. The term "best strain" is defined as any strain comprising said regulatable gene expression construct and which can survive in growth inhibiting conditions.

The individual genes that together form the library or population of potential biocatalysts may be encoded on a plasmid, or chromosomal or episomal DNA.

The genes that together form the library or population of potential biocatalysts may be expressed constitutively; or they may be on an inducible expression vector, in which case they are expressed as a result of activation through one of various possible operably linked regulatory elements. In fact it is not limiting how and if the biocatalyst expression is induced. Expression may be constitutive, it might be under control of a native regulator, either known or not yet known, or it might be part of a library under control of a specifically designed induction system. Regardless of the way in which the biocatalyst gene(s) is/are expressed, it is preferred that expression is sufficient to allow proliferation of the host cell.

In a further preferred embodiment of the above-described aspects at least one micro-organism host cell may comprise one (or more) nucleic acid molecule(s) encoding at least two potential biocatalysts, each capable of catalyzing at least one reaction of a multi-step chemical conversion reaction. Preferably, the product of the reaction catalyzed by one potential biocatalyst is the substrate for the reaction catalyzed by another potential biocatalyst, wherein at least one of said products is an effector compound for switching the riboswitches. In this way complete biochemical pathways may be uncovered and specific biocatalysts selected for each reaction step. In another embodiment, nucleic acid molecules encoding the various potential biocatalysts may be provided in separate expression systems, each expression system facilitating the expression of separate potential biocatalysts. Also in this manner, multi-step chemical conversion reactions may be provided for.

The host organism may or may not itself comprise enzymes and optional co-factors for catalysing a multi-step reaction in which the substrate is converted into a final intermediate compound, which said final intermediate compound is then converted into the desired product by a potential biocatalyst. The final intermediate compound may be a natural intermediate in the host cell that is produced by an available enzyme or a set of enzymes which is/are native to the host cell. Alternatively, the potential biocatalyst may produce an intermediate compound, which may or may not be an effector compound for switching the riboswitches, and which can be converted into a product by an enzyme naturally encoded for and expressed (i.e. available) in the host organism under the conditions provided. Thus, the potential biocatalyst need not necessarily catalyze the final reaction in a multi-step reaction process. It should be noted that in such instances, the enzyme system present in the host cell that contributes towards forming the effector compound for switching the riboswitch in such a multi-step system, may be encoded on host cell chromosomal or episomal DNA, including possibly plasmid DNA that also encodes the regulatable gene expression construct itself. Thus, apart from potentially catalysing the final reaction towards formation of the product, the potential biocatalyst as defined herein may also catalyze the critical reaction in a possible chain of reactions of a multi-step reaction process, which critical reaction is the sought after reaction that ultimately leads (possibly via additional reaction steps) to the production of the sought after product.

Thus, the host organism may or may not comprise one or more nucleic acid molecules encoding at least two biocatalysts each capable of catalyzing at least one reaction of a multi-step chemical conversion reaction.

In yet another preferred embodiment of the above-described aspects any host cell identified as comprising a biocatalyst may or may not gain an increase in biocatalytic activity by genome engineering of the host genome. Methods for genome engineering are known to persons skilled in the art and include, but are not limited to, multiplex genome engineering (MAGE), mutator strains, chemical mutagenesis, genome shuffling, and genome synthesis.

In yet another preferred embodiment of the above-described aspects the selection method may be used to identify a host cell or cells which survive in higher concentrations of the desired product, such that increased levels of production of the product will not result in the premature death of the host cell.

The chemical conversion reaction may be any chemical reaction which benefits from activation or acceleration by a biocatalyst. Reaction types that can be catalyzed by the potential biocatalyst include, but are not limited to, reactions catalysed by each of the major enzyme classes and subclasses: oxidoreductases, transferases, hydrolases, lyases, isomerases and ligases.

### Transporter Compound

The term "transporter compound" is used herein to refer to a primary modulator compound that transports an effector compound into the host micro-organisms. The production of a "transporter compound" is illustrated in Figure 3 Route **B:** the cloned DNA fragment, 1, is expressed producing the primary modulator compound, 5, which transports an effector compound, 4, into the host cell.

In many instances, the transporter compound will be an enzyme, however, it may also be a complex of enzymes, a combination of an enzyme and a co-factor, an RNA molecule, etc. The term is intended to cover all forms of the compound(s), both in the form of a protein, possibly modified by covalent or non-covalent attachment of sugars, oligosaccharides, polysaccharides or fatty acids, lipids, steroids and other chemical substituents and compounds, in the form of a nucleic acid or in the form of a combination of several such compounds. Generally, the true nature of the transporter compound may first be established or assessed after its selection using the method of the present invention.

The population of potential transporter compounds from which a transporter compound is to be selected in the method of the present invention is very suitably provided in the form of a library of nucleic acid molecules. Such nucleic acid molecules may comprise much more information than that encoding the transporter compound itself. However, this is of no consequence to its possible selection. A "library" can be produced by digesting all DNA from a suitable source organism with a restriction enzyme or by sonication and cloning the fragments into a vector, which will result in the production of many different recombinant vectors, each with a different fragment of DNA cloned into it. The collection of many different recombinant vectors together forms the "library". The library may be produced by any method available to the skilled person, for instance by shotgun cloning. "Shotgun cloning" is referred to as using the whole genome of an organism as the starting point for cloning. Alternatively, DNA/RNA can be isolated from the environment, resulting in thousands of different genes which may have derived from many different organisms; such a collection of nucleic acid material is referred to as a metagenome. At the other end of the spectrum, specific libraries may be constructed that contain only genes expected to encode the desired transporter compounds, based for instance on DNA homologies, previous screening experiments, or commercially available libraries. A library constructed by shotgun cloning techniques may contain from a few thousand (microorganisms) to hundreds of thousands (higher eukaryotes or microbial metagenomes) of different recombinant plasmids while only one or a few comprise a sequence of interest. In order to reduce the size of libraries derived from eukaryotic organisms, it is sometimes advantageous to produce cDNA libraries. Since protein coding regions generally account for only a small percentage of the total genome size of eukaryotic organisms and since eukaryotic cells generally express only a subset of their genes, the production of cDNA copies of the cellular mRNA using reverse transcriptase and the cloning thereof into a cDNA library results in a library of reduced size comprising only the part of the organism's genome that is of most interest (the protein coding sequences that are expressed). cDNA library construction is well known to the skilled person. Other library types include, but are not limited to, a library obtained by i) mutation (directed or random) of a single parent gene ii) recombination of several parent genes (such as libraries produced by gene shuffling) iii) a combination thereof.

The "library" may be a library of genes or a library of organisms.

The individual genes that together form the library or population of potential transporter compounds may be encoded on a plasmid, or chromosomal or episomal DNA.

The genes that together form the library or population of potential transporter compounds may be expressed constitutively; or they may be on an inducible expression vector, in which case they are expressed as a result of activation through one of various possible operably linked regulatory elements. In fact it is not limiting how and if the transporter compound expression is induced. Expression may be constitutive, it might be under control of a native regulator, either known or not yet known, or it might be part of a library under control of a specifically designed induction system. Regardless of the way in which the transporter compound gene(s) is/are expressed, it is preferred that expression is sufficient to allow proliferation of the host cell.

In a preferred embodiment of the above-described aspects at least one micro-organism host cell may comprise one (or more) nucleic acid molecule(s) encoding at least two potential primary modulator compounds, wherein at least one primary modulator compound is a biocatalyst (as used herein) and at least one primary modulator compound is a transporter compound, wherein the substrate of the reaction catalyzed by said biocatalyst is transported into the cell by said transporter compound.

### The host cell

The host cell of the present invention may be any host cell capable of providing the cellular transcription and translation machinery required for expression of the population of potential primary modulator compounds and of the necessary components of the regulatory gene expression construct.

The host cell may be, but is not limited to, a micro-organism, such as a prokaryotic micro-organism or a eukaryotic micro-organism, a plant cell, or an animal cell line. Suitable prokaryotes include both archaea and bacteria. Suitable eukaryotic micro-organisms include yeast cells, fungi or protist cells. Suitable animal cell lines are for instance insect cell lines or cells of birds, reptiles, and fish, but also mammalian (including human) cell lines may be used.

### The substrate

The term "substrate" as used herein refers to the reactant in the "biocatalyst" catalyzed reaction, i.e. the chemical compound that interacts with the active site of the primary modulator compound and is converted to a product, preferably directly into the effector compound.

The substrate may or may not be a product of another intracellular biocatalysis reaction or series of reactions, wherein said reaction(s) are performed by biocatalysts encoded in the genetic material present in the host cell.

In order for a substrate added to the cell's external environment to come into contact with the potential biocatalyst, the host cell may comprise a specialized or general uptake system for transporting the substrate across the cell membrane in order to take up the substrate.

However, this is not essential as simple diffusion of the substrate across the cell membrane into the intracellular environment is also envisioned. Substrate transport through cellular membranes depends on the chemical properties of the substrate itself (e.g. hydrophobicity, ionic character) and the permeability of the host cell membrane. It should be understood that if a "biocatalyst" is produced in a certain cell compartment, it is essential that the substrate can be in direct physical contact with said "biocatalyst" so that conversion into the product can take place.

The initial substrate used in the sought after biocatalyst conversion reaction for preparing the product compound generally are either available from commercial suppliers or may be prepared by methods known to the skilled person.

### Positive Selection

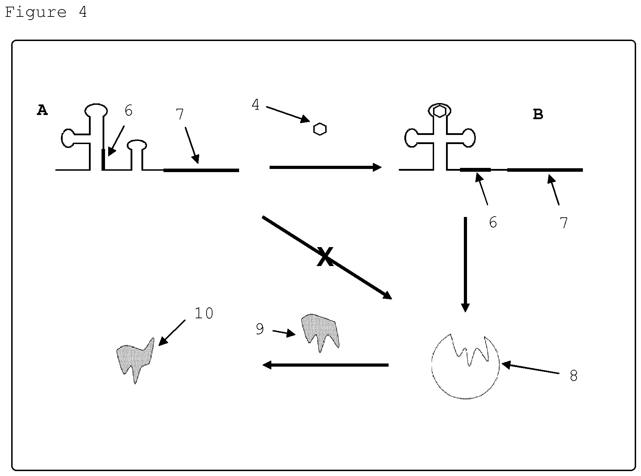

The general mechanism by which positive selection functions is illustrated in Figure 4. Note: Figure 4 illustrates only one of the two regulation sequences. The riboswitch A in the unbound form conceals its associated riboswitch binding site (RBS), 6. This disallows expression of the coding region, 7, thus the secondary modulator compound, 8, is not available to modulate the action of the growth regulator compound, 9, and therefore the cell fails to proliferate. The riboswitch **B,** wherein the effector compound, 4, is bound, undergoes a conformational change such that the RBS is exposed. This allows the RBS to bind to the ribosome and express the coding region, 7, such that the secondary modulator compound, 8, is expressed. Expression of the secondary modulator compound, 8, modulates the action of the growth regulator compound, 9, such that it becomes a non-growth inhibiting compound, 10, and the cell proliferates.

In a preferred embodiment, a method for detecting a primary modulator compound according to the positive selection mechanism may essentially be performed as follows:
A multitude of micro-organism host cells is provided, each comprising a DNA fragment or fragments which may encode a desired primary modulator compound or compounds and each provided with a regulatable gene expression construct as described herein, in this case each riboswitch is an 'ON' riboswitch and each operably linked coding region encodes a secondary modulator compound which confers antibiotic resistance, expression of which produces an enzyme capable of deactivating the respective antibiotic.

The host cells are introduced in a growth medium, comprising essential growth nutrients and two antibiotic compounds at a level sufficient to inhibit the growth of cells not capable of expressing the antibiotic resistance gene.

The growth medium may comprise a test substrate(s) which can be converted into, or which may itself be, an effector compound or compounds which can switch/activate the riboswitch. The growth medium may further comprise one or more inducer compounds for initiating the expression of one or more biocatalysts inside the host cells in case their expression is inducible. The induction of one or more primary modulator compound expression systems need not be continuous, but may also be discontinuous to the extent that expression is needed.

Cells that express a suitable primary modulator compound will start to overcome the antibiotic growth inhibition and proliferate, while growth of the remaining cells will remain inhibited by the antibiotic compounds.

The composition of the growth medium is determined by the host cell requirements. The concentration of the growth regulator compounds in the growth medium will each generally range between 1 µM and 1M. Suitable concentrations can easily be determined by the skilled person. The method involving the host cells will generally be performed at a host cell-specific temperature, which in the case of micro-organisms may be in the range of about 20°C-37°C.

In another preferred embodiment, a method for detecting a primary modulator compound according to the positive selection mechanism may be performed as described above, except that one of said coding regions is a gene that complements an auxotrophy in the host cell, and expression of said gene produces a secondary modulator compound which converts a non-essential growth compound in the growth medium into an essential growth compound which can be utilised by said auxotrophic host cell, and thus said host cell proliferates. An example of a gene that complements such an auxotrophy in the host cell is the E. coli thrC gene, encoding a threonine synthase enzyme. Said enzyme converts O-phospho-L-homoserine into L-threonine. If the thrC gene is not expressed then the host cell does not convert O-phospho-L-homoserine into L-threonine and thus the cell fails to proliferate even in the presence of O-phospho-L-homoserine.

### Negative Selection

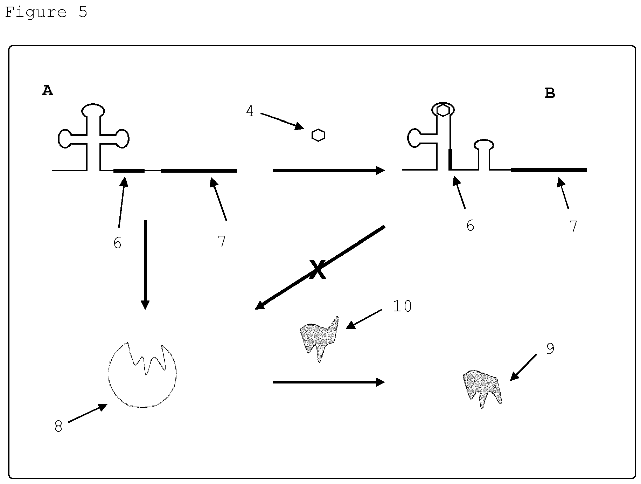

The general mechanism by which negative selection functions is illustrated in Figure 5. Note: Figure 5 illustrates only one of the two regulation sequences. The riboswitch, A, in the unbound form exposes its RBS, 6, which consequently allows expression of its coding region, 7, to form the secondary modulator compound, 8. The secondary modulator compound, 8, converts a non-cytotoxic compound, 10, into a growth regulator compound, 9, and the cell fails to proliferate. The riboswitch B, wherein the effector compound, 4, is bound, undergoes a conformational change such that the RBS, 6, is concealed. This disallows expression of the coding region, 7, which consequently stops the production of the secondary modulator compound, 8. In the absence of the secondary modulator compound, 8, the non-growth inhibiting compound, 10, is not converted into the growth regulator compound, 9, and the cell proliferates.

In a preferred embodiment, a method for detecting a primary modulator compound according to the negative selection mechanism may essentially be performed as follows:
A multitude of micro-organism host cells is provided, each comprising a DNA fragment or fragments which may encode a desired primary modulator compound or compounds and each provided with a regulatable gene expression construct as described herein, in this case each riboswitch is an 'OFF' riboswitch and each operably linked coding region encodes a secondary modulator compound, expression of which produces an enzyme which converts a non-cytotoxic compound into a growth regulator compound.

The host cells are introduced in a growth medium, comprising essential growth nutrients and a non-cytotoxic compound capable of being converted into a growth regulator compound by said secondary modulator compound.

The growth medium may comprise a test substrate(s) which can be converted into, or which may itself be, an effector compound or compounds which can switch/activate the riboswitch. The growth medium may further comprise one or more inducer compounds for initiating the expression of one or more biocatalysts inside the host cells in case their expression is inducible. The induction of one or more primary modulator compound expression systems need not be continuous, but may also be discontinuous to the extent that expression is needed.

Cells that express a suitable primary modulator compound will start to overcome the growth suppression which is caused by expression of the coding region.

The composition of the growth medium is determined by the host cell requirements. The concentration of the non-cytotoxic compounds in the growth medium will each generally range between 1 µM and 1M. Suitable concentrations can easily be determined by the skilled person. The method involving the host cells will generally be performed at a host cell-specific temperature, which in the case of micro-organisms may be in the range of about 20°C-37°C.

### Positive and Negative selection

Once the primary modulator compound is selected, it may be isolated and optionally purified by methods known in the art. Alternatively, the selected genetic coding sequence encoding a primary modulator compound is introduced into a micro-organism host cell by transgenic methods known in the art, thereby producing a production strain. The term "production strain" means the host cell wherein the primary modulator compound is produced at high levels. The production strain may produce the primary modulator compound intracellularly or may secrete said primary modulator compound, optionally as a fusion protein. The primary modulator compound can be isolated from the growth medium, optionally after removal of the cells. Additional processing, such as purification of the primary modulator compound may be necessary.

Should the primary modulator compound be a biocatalyst it may be used in a method for the manufacture of a desired product, simply by allowing it to convert a substrate under suitable reaction conditions. Conversion generally will take place in a reaction mixture comprising the biocatalyst and one or more substrates and, if needed, cofactors for the biocatalytic conversion reaction, optionally in the presence of additional compounds, such as buffers, chelators, ions or reducing compounds supporting the proper conformational folding of the enzyme.

The biotransformation of a substrate into a desired product can be performed by using permeabilized cells, resting cells, growing cells, or a combination of these as biocatalysts. The biotransformation can be performed by using crude cell extracts as biocatalysts. The biocatalysts can be immobilized on or in a water insoluble carrier or support system. The biotransformation can be performed in aqueous medium. It can also be performed in multi-phasic media possibly comprising two or more of the following: a solid phase, an aqueous phase, an organic phase, or a gaseous phase. Depending on the characteristics and purity of the enzyme, a sufficient time is allowed for the biotransformation reaction to proceed, after which the product or products and remaining substrate or substrates and cofactors may be recovered from the reaction mixture. Reaction conditions generally depend on the temperature optimum of the enzymatic reaction. Upon recovery, the product, remaining substrates and cofactors may be further purified by methods known in the art.

### Examples

### Materials and general considerations

Plasmid DNA and PCR products were purified using QIAprep® Spin Miniprep Kit and QIAquick® PCR Purification Kit respectively (purchased from Qiagen). Gel extractions were performed using GeneJET™ Gel Extraction Kit (purchased from Fermentas). Caffeine, theophylline, theobromine, xanthine and all antibiotics used herein were purchased from Sigma-Aldrich. Synthetic oligonucleotides were purchased from Integrated DNA Technologies. The following examples utilised Escherichia coli Dh10B host cells and culture experiments were performed in Luria Broth (LB) or M9 minimal media supplemented with 0.1 g/L leucine and trace metals (known in the art and available from numerous suppliers). All strains were kept at -80 °C in a 15 % v/v glycerol solution. Plasmid manipulations were performed using USER cloning as previously described (Bitinaite & Nichols, 2009, herein referred to in its entirety). Sequences of all vectors constructed were verified by DNA sequencing (sequencing performed by Macrogen, The Netherlands or Beckman Coulter, UK).

### Primers used

Underlined sequences will be removed, generating a single stranded overhang during USER cloning.

| **Primer** | **Sequence (5'-3')** | **SEQ ID NO** |
|---|---|---|
| GEBO1 | ATTCTCTAGAAAGUATAGGAACTTCTCTAAGAAACCATTATTATCATG | 1 |
| GEBO2 | ACTTTCTAGAGAAUAGGAACTTCCGAAAGGGCCTCGTGATACG | 2 |
| GEBO3 | ACTTCGGGUCCTCAACGACAGGAGCACGATCAT | 3 |
| GEBO4 | ACCCGAAGUTCCTATTCTCTAGAAAGTATAGGA | 4 |
| GEBO5 | AGTGATCTUCTTCTCCATCTTGTTGTTACCTTA | 5 |
| GEBO6 | AAGATCACUGGATATACCACCGTTGAT | 6 |
| GEBO29 | GGCGTATCACGAGGCCCTTTCG | 7 |
| GEBO31 | CCGGGCTGCAGGAATTCGAT | 8 |
| GEBO42 | AAGTTTACUCGAACTTCCATATGAGGCC | 9 |
| GEBO43 | ACTCCGCUAGCGCGCCGAACGACCGAGCGCA | 10 |
| GEBO44 | AGCGGAGUGTATACTGGCTTACTATGTTGG | 11 |
| GEBO45 | ATGGACAACUTATATCGTATGGGGCTGA | 12 |
| GEBO46 | AGTTGTCCAUATGAATATCCTCCTTAGGAAT | 13 |
| GEBO47 | AGTAAACTUGGTCTGACAGTTACCAATG | 14 |
| GEBO72 | ACAAGAUGAGGGAAGCGGTGATCGC | 15 |
| GEBO73 | ATCTTGUTGTTACCTTAGCAGGGTGCT | 16 |
| GEBO74 | ATCCTGACGGUTGACGGCTAGCTCAGTCC | 17 |
| GEBO77 | ACCGTCAGGAUCAACGTTCAAATCCGCTC | 18 |

### Preparation of Electrocompetent E. coli cells

The following outlines the procedure for preparing electrocompetent cells as used in the following examples: (1) Inoculate a single E. coli colony in 10 mL LB medium; (2) incubate overnight at 37 °C with shaking; (3) transfer 0.5-1 mL of the culture to 250 mL fresh LB medium; (4) incubate at 37 °C for 3-4 hours (until OD₆₀₀ ≈ 0.4); (5) cool the cells in an ice/water bath for 15 minutes; (6) collect the cells by centrifugation (5-10 minutes, 4000 rpm, 2 °C - 185-190 g total weight per tube); (7) wash 2 times in water - collect cells by centrifugation (5-10 minutes, 4000 rpm, 2 °C - 185-190 g total weight per tube); (8) add water to final volume of 1-2 mL; (9) aliquot cells in ice-cold eppendorf tubes (50/100 µL in each).

### Electroporation of electrocompetent E. coli cells

The following outlines the electroporation procedure for electrocompetent E. coli cells as used in the following examples: (1) put cuvettes on ice 20 minutes before electroporation; (2) prepare falcon tubes with 2 mL prewarmed SOC medium; (3) thaw electrocompetent E. coli cells on ice; (4) add 1-2 µL (≤400 ng) DNA to each sample; (5) transfer the cell/DNA mixture to the cuvettes; (6) electroporate at 2.5 kV; (7) suspend the cells in prewarmed SOC medium immediately after electroporation; (8) allow for phenotypic expression at 37 °C (minimum 1 hour at 200 rpm); (9) plate 1 µL of recovered cells on LB/agar plates containing kanamycin (50 µg/mL) and ampicillin (50 µg/mL) to determine success of transformation; (10) transfer SOC medium to 9 mL LB liquid medium containing kanamycin (50 µg/mL) and ampicillin (50 µg / mL). Grow onto stationary phase.

### Example 1. Construction of the dual-riboswitch selection vector pGEB05.1

pGEBO5.1 is a third generation selection vector based on the second generation selection vector pGEBO4.1, and pGEBO4.1 is based on the first generation selection vector pGEBO4. pGEBO4 comprises the vector pGEBO2 which contains the 'Riboswitch Platform'.

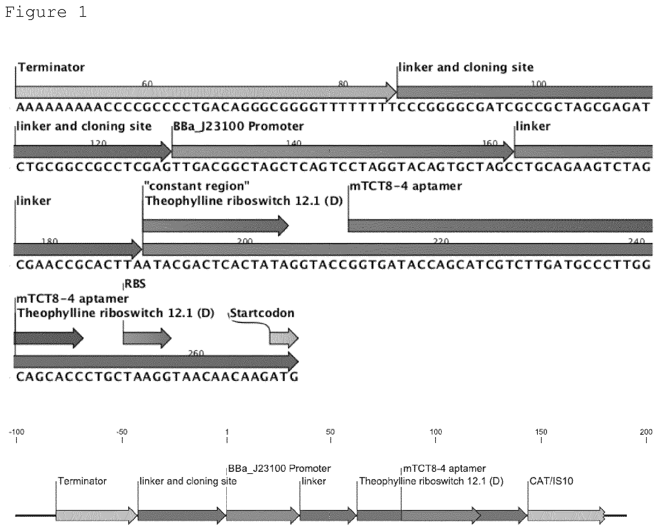

Construction of pGEBO2: the 221 base pair DNA sequence that constitutes the 'Riboswitch Platform' (Figure 1) was artificially synthesised by Integrated DNA Technologies and delivered on a pIDTSMART vector yielding pGEBO2. In addition to the riboswitch platform a Flippase Recognition Target (FRT) sequence (5'-GAAGTTCCTATTCTCTAGAAAGTATAGGAACTTC-3' (SEQ ID NO: 19)) was included 5' to the riboswitch.

Construction of pGEBO4: pGEBO4 was created by circular USER fusion of three PCR products: (1) the FRT-riboswitch platform amplified from pGEB02 with primers GEBO4/5 (forward/reverse); (2) a region containing a chloramphenicol acyltransferase (cat), pMB1 origin of replication (ori) and ampR (ampicillin selection marker) amplified from pSKD314 (Gallivan, 2004) with primers GEBO6/2; and (3) tetR (tetracycline selection marker) amplified from pBR322 (Sutcliffe, 1978) with primers GEBO1/3. Primers were designed such that that three fragments were fused seamlessly in a circle in the order (1)-(2)-(3). Primer GEBO2 and GEBO1 each included a part of a FRT sequence in the primer-tails such that fusion of fragment (2) and (3) resulted in a full FRT sequence between (2) and (3) in the final construct. In this way tetR becomes flanked by two FRT sites for eventual removal/exchange by recombineering (Schlake & Bode, 1994).

Construction of pGEBO4.1: pGEBO4.1 was created by circular fusion of the following three fragments: (1) Riboswitch platform, cat amplified from pGEBO4 with primers GEBO42/43; (2) p15A ori amplified from pACYC184 (Li & Tu, 1991) with primers GEBO 44/45; and (3) ampR and specR amplified from pSLY47 (Lemire et al., unpublished, details available from applicant on request) with primers GEBO46/47. The fragments were circular USER fused in the order (1)-(2)-(3).

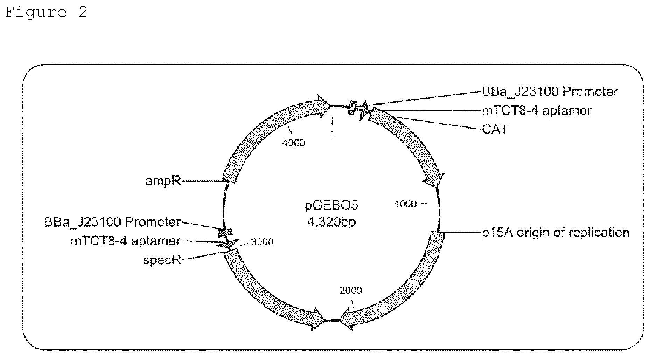

Construction of pGEBO5.1: pGEBO5.1 was created by circular USER fusion of two fragments, both amplified from pGEBO4.1: (1) Riboswitch platform (from promoter to start codon) with primers GEBO73/74; and (2) the entire pGEBO4.1 backbone excluding 5' UTR and promoter of specR with primers GEBO77/72. The fusion of (1) and (2) resulted in a new vector with an additional riboswitch platform directionally fused to control specR expression (see figure 2).

### Example 2. Determining spontaneous resistance in single and double selection systems

pGEBO5.1 was transformed into E. coli Dh10B cells (forming EcpGEB05.1) using standard methods known to the skilled person. To test whether the double selection system solved the problem of spontaneous resistance, plating of up to 1.3x10⁸ EcpGEBO5.1 cells was performed in 10 replicates on LB agar media containing spectinomycin (80 µg / mL) and chloramphenicol (30 µg / mL) ('dual selection'). As a control, plating on plates containing only chloramphenicol (30 µg / mL) or spectinomycin (80 µg / mL) ('single selection') was each performed in triplicate (See Table 1). Each plate was incubated for 72 hours. Of the 10 plates with dual selection no colonies were observed within the first 48 hours of incubation. In 24 h of incubation, each of the three plates containing only chloramphenicol produced numerous colonies, as did plates containing only spectinomycin. Spontaneous resistance was only observed in the dual selection system when plating 1.3×10⁸ cells and only after 72 h of incubation, whereas in the single selection systems spontaneous resistance was observed for all cell densities within a short time frame. Therefore, it appears that the probability of a single mutation to confer spontaneous resistance to one antibiotic is significant whereas the probability of a simultaneous double mutation to confer spontaneous resistance to both antibiotics is effectively nil. Thus, the level of false positives as a result of spontaneous resistance is effectively reduced to zero through the use of the dual selection system.

**Table 1. '-' no growth, '+' single colonies (1-1000), '++' confluent layer. Each selection system performed in triplicate; x,y,z: x = 24 h growth, y = 48 h growth, z = 72 h growth.**

| **Volume of overnight culture plated** | **100 ul** | **10 ul** | **1 ul** |
|---|---|---|---|
| Number of CFU (cell forming units) plated: | 1.3×10⁸ | 1.3×10⁷ | 1.3×10⁶ |
| chloramphenicol 30 µg / mL | ++,++,++ | +,++,++ | -,+,+ |
| spectinomycin 80 µg / mL | ++,++,++ | +,++,++ | +,++,++ |
| chloramphenicol 30 µg / mL + spectinomycin 80 µg / mL | -,-,+ | -,-,- | -,-,- |

### Example 3. Growth response of EcpGEB05.1 for different xanthine alkaloids

The growth response of EcpGEBO5.1 for 4 different xanthine alkaloids was determined empirically using a selection assay with varying concentrations xanthine-based compounds: theophylline, xanthine, theobromine and caffeine.

Procedure: 300-400 colony forming units were plated on plates containing ampicillin (50 µg / mL), spectinomycin (80 µg / mL), chloramphenicol (30 µg / mL) and the indicated concentration of xanthine-based compound - see Table 2: Dissociation constants (K_{d}) for the xanthines with the aptamer region (mCTC8-4 aptamer) previously determined (Jenison et al., 1994). No selection means plates without spectinomycin and chloramphenicol. (+) means growth was observed. (-) means no growth was observed. (∼) means reduced growth was observed.

**Table 2 - specificity characterisation of the riboswitch aptamer domain in pGEBO5.1.**

| **Xanthine** | K_{d} (µM) | No Selection | Selection |
|---|---|---|---|
| **Theophylline** | | | |
| 2 mM | 0.32 ± 0.13 | + | + |
| 0.1 mM | | + | ∼ |

| **Xanthine** | | | |
|---|---|---|---|
| | 8.5 ± 0.40 | | |
| 1 mM | | + | - |

| **Theobromine** | | | |
|---|---|---|---|
| | > 500 | | |
| 1 mM | | + | - |

| **Caffeine** | | | |
|---|---|---|---|
| 7 mM | 3500 ± 1500 | ∼ | - |
| 3 mM | | + | - |

These results show that cells containing a regulatable gene expression construct with riboswitch aptamer domains specific for theophylline will only proliferate under selection conditions when theophylline is present in the growth medium.

The lack of growth in media containing xanthine was initially surprising considering the dissociation constant for xanthine with the theophylline aptamer region (K_{d} = 8.5 ± 0.40 µM), however, as discussed below, this was attributed to a lack of membrane permeation of xanthine from the extracellular to intracellular environments.

### Example 4. Riboswitch based functional selection of metagenomic libraries for riboswitch activating genes.

57SDb01 (Sommer, Dantas & Church, 2009) and 57SDb03 (Sommer et al.,2009) are human faecal metagenomic DNA libraries and AB95D01 is a soil metagenomic DNA library. Briefly, each of the libraries were prepared by extracting DNA from the above mentioned environments, shearing of the DNA into 1-3 kb sizes and cloning this DNA into the pZE21 cloning vector (Lutz & Bujard, 1997; cloning vector with no insert). Electrocompetent EcpGEBO5.1 cells were prepared using the method as described above. 400 ng of plasmid DNA from each of the three libraries, 57SDb01, 57SDb03 and AB95D01 as well as 2 ng of pZE21 as control was transformed into electrocompetent EcpGEBO5.1 cells by electroporation, using the method as described above. Following recovery for 1 hour at 37 °C in 1 mL SOC medium, the cultures were transferred to 9 mL of LB media containing kanamycin (50 µg/mL) and ampicillin (50 µg / mL) and grown at 37 °C with shaking until stationary phase. For each metagenomic library 50 and 100 µL corresponding to 0.5 - 1 x 10⁸ cells was plated on LB/agar (dried for ∼ 2 hours) plates containing ampicillin (50 µg / mL), kanamycin (50 µg / mL), chloramphenicol (30 µg / mL), spectinomycin (80 µg / mL) and incubated at 37 °C for 69 hours. The LB/agar was also found to contain trace quantities of xanthine. The remaining cultures that were not plated were kept at -80 °C in a 15% glycerol solution for subsequent screening.

After 69 hours at 37 °C, 41 colonies had appeared on the plates plated with the EcpGEBO5.1 transformed with the AB95D01 library, while 3 colonies had appeared on the plates plated with EcpGEBO5.1 transformed with the 57SDb01 library. Zero colonies appeared after 69 hours on plates plated with EcpGEBO5.1 cells transformed with the control vector pZE21 or the 57SDb03 library. To confirm resistance of the selected clones, each of the colonies were restreaked and grown on selective medium. Resistance was confirmed for all strains except two colonies selected from the 57SDb01 library that were sensitive and did not grow in the confirmation experiment.
Each of the colonies were inoculated in LB medium containing ampicillin (50 µg/ml) and kanamycin (50 µg /ml) and incubated at 37 °C for 20 h. The metagenomic insert of each of the selected colonies was sequenced bidirectionally with primers GEBO29/31 by Sanger sequencing (Beckman Coulter, UK). For each read, low quality score data (<0.015, ambiguous nucleotides > 2) as well as sequence matching the cloning vector pZE21 was trimmed and excluded for further analysis using CLC DNA Workbench 6.1. Assembly of each pair of reads from each clone was performed with the same software. Using Blastx, the assembled sequences were compared to the GenBank non redundant protein sequence database (January 19, 2013). Blastx computes the local sequence alignment between the nucleotide query translated in all 6 frames and the non-redundant nucleotide database translated in all 6 frames.

Based on this, the function of each of the identified open reading frames was hypothesized (See Table below). The sequence analysis identified 35 clones harbouring an identical metagenomic insert sequence with origin from the AB95D01 library encoding a putative multidrug transport protein with a 79% identity to a protein from *Pseudomonas* species. Another insert was found to encode another putative multidrug efflux protein with 99% identity to a protein from *Serratia liquefaciens.* If multidrug export targets both chloramphenicol and spectinomycin it is one of the mechanisms that can effectively overcome a dual selection, and hence these results were expected.

**Table 3.**

| | **Blastx** | | | | **Putative mechanism for enabling growth selection** | **No. of colonies with insert** |
|---|---|---|---|---|---|---|
| | **Description (top e-value)** | **e-value** | **hit-length** | **Phylogenetic origin** | | |
| **AB95D01 _insert 1* ∼2.5-3 kb** | | | | | | |
| ORF1 | multidrug/solvent transporter | 0.0 | > 978 bp | *Pseudomonas syringae* | Multidrug efflux protein conferring antibiotic resistance towards both spectinomycin and chloramphenicol | 35 |
| ORF2 | hypothetical protein | 3.02E-25 | 264 bp | *Serratia marcescens* | | |

| **AB95D01 insert 2 * ∼2.5-3 kb** | | | | | | |
|---|---|---|---|---|---|---|
| ORF1 | MFS multidrug transporter | 9.17E-125 | 804 bp | *Serratia liquefaciens* | Multidrug efflux protein conferring antibiotic resistance towards both spectinomycin and chloramphenicol | 2 |
| ORF2 | alanine racemase | 0.0 | 957 bp | *Serratia liquefaciens* | | |

| **AB95D01 _Insert 3 1563 bp** | | | | | | |
|---|---|---|---|---|---|---|
| ORF1 | Xanthine permease | 0.0 | 1317 bp | *Acinetobacter baumannii* | Increased intracellular xanthine concentration from import | 2 |

| **57SFB01 _Insert 4, 2259 bp** | | | | | | |
|---|---|---|---|---|---|---|
| ORF1 | Xanthine permease | 0.0 | 1416 bp | *Prevotella copri* | Increased intracellular xanthine concentration from import | 1 |

| | | | | | | |
|---|---|---|---|---|---|---|
| * bidirectional sequencing did not cover the full insert, so the sequence is not known. | | | | | | |

Two sequences were identified that had high similarity (99%) to xanthine permeases (enzymes that transport xanthine across a cell membrane). One was found among the clones selected from the AB95D01 soil library (SEQ ID NO: 20) and one from the 57SDb01 faecal library (SEQ ID NO: 21). As mentioned in Example 3, xanthine has been shown to bind the theophylline riboswitch aptamer (k_{d} = 8.5 ± 0.40 µM), but previously the addition of 1 mM xanthine to the growth medium did not enable growth of EcpGEBO5.1 under selective conditions. Presumably, this was due to a low degree of import of xanthine by EcpGEB05.1. As mentioned above, LB media (in which the selection experiment was performed) contains trace quantities of xanthine. Coupling this to the presence of a xanthine permease in the proliferating cells, it is likely that the intracellular concentration of xanthine rises to levels that activate the riboswitch, thus we hypothesized that this was the mechanism by which the selected xanthine permeases enabled growth during selection.

In order to test the hypothesis, growth experiments with and without xanthine was performed. First, in order eliminate the putative contribution of any background mutation of the genome or of the pGEBO5.1 assay plasmid the library plasmid encoding the putative xanthine permease (pZE21_1H) from the AB95D01 soil metagenomic library was extracted and retransformed in strain EcpGEB05.1. Instead of testing the strain in LB-medium, xanthine-free M9 minimal medium was used to test the effect of defined xanthine concentrations. Strain EcpGEBO5.1 transformed with the empty cloning vector pZE21 (EcpGEB05.1 + pZE21) was used as a control. Both strains were inoculated in 3 ml M9 minimal medium supplemented with kanamycin 50 µg/ml and ampicillin 50 µg/ml and grown overnight. The next day a new 3 ml culture was prepared under same conditions and inoculated by transferring 30 µl from the initial culture. At exponential phase (when the optical density at 600 nm (OD600) had reached ∼0.5) the culture was used to inoculate 200 µl M9 minimal selective media in a microtiterplate with varying concentrations of xanthine (Table below). Inoculums were diluted such that the initial OD600 of each culture was exactly 0.01. The microtiter plate was incubated at 37 °C in an ELx808 Absorbance Microtiterplate Reader from Biotek with shaking at 200 rpm. OD600 was measured after 16 h and the average and standard deviation was calculated from three biological replicates of each xanthine concentration. When grown in concentrations of xanthine ranging from 0 mM to 1 mM but no chloramphenicol or spectinomycin both strains were unaffected by the presence of xanthine. When applying selection (chloramphenicol 30 µg /ml and spectinomycin 80 µg /ml), growth was observed only for the strain harbouring the xanthine permease containing plasmid (pZE21_1H) and only when xanthine was added (Table 3). The experiment confirms the prediction that the DNA insert is encoding a xanthine permease protein, and that the increased xanthine uptake enables growth under selective conditions.

**Table 3. Growth response to varying xanthine concentrations.**

| Xanthine (µm) | OD₆₀₀ of EcpGEBO5.1 + pZE21 | OD₆₀₀ of EcpGEBO5.1 + pZE21_1H |
|---|---|---|
| 0 | 0.77 ± 0.01* | 0.71 ± 0.02* |
| 0 | 0.01 ± 0.00 | 0.00 ± 0.00 |
| 1.0 | 0.02 ± 0.00 | 0.17 ± 0.01 |
| 1.5 | 0.02 ± 0.00 | 0.29 ± 0.01 |
| 2.0 | 0.02 ± 0.00 | 0.37 ± 0.00 |
| 2.5 | 0.03 ± 0.00 | 0.42 ± 0.00 |
| 4.5 | 0.03 ± 0.00 | 0.50 ± 0.01 |
| 12.5 | 0.03 ± 0.01 | 0.51 ± 0.01 |
| * no selection applied | | |

This example clearly demonstrates that the herein described gene selection system can be used to select from a vast metagenomic library a gene sequence encoding transporter enzyme for transporting an effector compound, in this instance xanthine, from the extracellular space into the cell cytoplasm.

It should be understood that this invention is not limited to selecting transporter compounds, and that biocatalysts can be selected using the method for selecting a transporter compound as demonstrated in Example 4.

In brief, a precursor compound (i.e. a substrate that is to be bio-transformed into a desired product) can be fed to a competent cell line comprising the regulatable gene expression construct (e.g. pGEB05.1) which has been transformed with a metagenomic library, as described above. Cells that subsequently proliferate under selection conditions can then be selected and sequenced to determine which comprise gene sequences encoding a biocatalyst for effecting the transformation of the precursor compound to a riboswitch aptamer binding compound (i.e. the desired bio-transformed product).

Said biocatalyst activity can then be verified in much the same manner as the verification process for the transporter compound as described above (i.e. grow the cells in the presence and absence of said precursor, and monitor cell growth).

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

1. Aharoni A, Griffiths AD, Tawfik DS. High Throughput Screens and Selections of Enzyme-Encoding Genes. Curr. Op. Chem. Biol. 2005; 9: 210-216.
2. Bitinaite J, Nichols NM. DNA Cloning and Engineering by Uracil Excision. Curr. Prot. Mol. Biol. 2009; Ch. 3; Unit 3.21.
3. Dietrich JA, McKee AE, Keasling JD. High Throughput Metabolic Engineering: Advances in Small Molevule Screening and Selection. Annual Rev. Biochem. 2010; 79: 563-590.
4. Gallivan JP, Desai SK. Genetic Screens and Selections for Small Molecules Based on a Synthetic Riboswitch that Activates Protein Tanslation. J. Am. Chem. Soc. 2004; 126: 13247-13254.
5. Jenison R, Gill S, Pardi A, Polisky B. High-Resolution Molecular Discrimination by RNA. Science 1994; 263: 1425-1429.
6. Kaeberlein T, Lewis K, Epstein SS. Isolating "Uncultivable" Microorganisms in Pure Culture in a Simulated Natural Environment. Science 2002; 296: 1127-1129.
7. Leemhuis H, Kelly RM, Dijkhuizen L. Directed Evolution of Enzymes: Library Screening Strategies. IUBMB Life 2009; 61: 222-228.
8. Li JK, Tu J. Ssp RFI, a Novel Class-II Restriction Endonuclease form Synechococcus RF-1 Recognizing 5'TT/CGAA-3'. Nucleic Acids Res. 1991; 19: 4470.
9. Lorenz P, Liebeton K, Niehaus F, Schleper C, Eck J. The Impact of Non-Cultivated Biodiversity on Enzyme Discovery and Evolution. Biocat. Biotrans. 2003; 21: 87-91.
10. Lorenz P, Eck J. Screening for Novel Industrial Biocatalysts. Eng. Life Sci. 2004; 4: 510-504.
11. Lutz R, Bujard H. Independent and Tight Regulation of Transcriptional Units in Escherichia coli via the LacR/O, the TET R/O and AraC/I1 - I2 Regulatory Elements. Pharmacia 1997; 25: 1203-1210.
12. Nestl BM, Nebel B, Hauer B. Recent Progress in Industrial Biocatalysis. Curr. Op. Chem. Biol. 2011; 15: 187-193.
13. Sanchez S, Demain AL. Enzymes and Bioconversions of Industrial, Pharmaceutical, and Biotechnological Abstract. Org. Proc. Res. Dev. 2011; 15: 224-230.
14. Sommer MO, Church GM, Dantas G. Functional Characterisation of the Antibiotic Resistance Reservoir in the Human Microflora. Science 2009; 325: 1128-1131.
15. Sommer MO, Church GM, Dantas G. A Functional Metagenomic Approach for Expanding the Synthetic Biology Toolbox for Biomass Conversion. Mol. Sys. Biol. 2010; 6: 360.
16. Steele HL, Jaeger KE, Daniel R, Streit WR. Advances in Recovery of Novel Biocatalysts from Metagenomes. J. Mol. Microbiol. Biotech. 2009; 16: 25-37.
17. Sutcliffe, JG. Nucleotide Sequence of the Ampicillin Resistance Gene of Escherichia coli Plasmid pBR322. Biochem. 1978; 75: 3737-3741.
18. Turner NJ. Directed Evolution Drives the Next Generation of Biocatalysts. Nat. Chem. Biol. 2009; 5: 567-573.
19. Yang J, Seo SW, Jang S, Shin S-I, Lim CH, Roh T-Y, Jung GY. Synthetic RNA devices to expedite the evolution of metabolite-producing microbes. Nature Communications 2012; 4: 1413.

## Claims

1. A method for the in-vivo selection of a genetic coding sequence encoding at least one primary modulator compound modulating the action of an effector compound, comprising growing an expression library of host micro-organisms in the presence of at least two growth regulator compounds each capable normally of preventing proliferation of said micro-organisms, which expression library expresses a library of DNA fragments potentially expressing said primary modulator compound, and selecting micro-organisms in said library which proliferate;
wherein said primary modulator compound
- effects a chemical transformation of a substrate into said effector compound and said substrate is provided to said microorganisms; or
- transports said effector compound into said micro-organisms, and
wherein said host microorganisms comprise a regulatable gene expression construct, said regulatable gene expression construct comprising a nucleic acid molecule, said nucleic acid molecule comprising at least two regulation sequences, each said regulation sequence encoding an RNA molecule comprising a riboswitch operably linked to a respective coding region, wherein each riboswitch regulates expression of its respective coding region in response to the presence of said effector compound, and each coding region encodes a respective secondary modulator compound for modulating the action of a respective said growth regulator compound, and wherein said at least two regulation sequences encode different secondary modulator compounds that act against different said at least two growth regulator compounds;
whereby a micro-organism in said library which produces a desired primary modulator compound is enabled to proliferate in the presence of said at least two growth regulator compounds.

2. A method as claimed in claim 1, wherein the expression library of host micro-organisms comprises a multitude of host cells that produces a population of potential primary modulator compounds, wherein said multitude of host cells:
- is a library of cells of a single cell type wherein essentially each host cell comprises a cloned nucleic acid fragment or mutated native genome encoding at least one potential primary modulator compound; or
- are cells of different cell types wherein essentially each host cell comprises a native nucleic acid fragment encoding at least one potential primary modulator compound.

3. A method as claimed in claim 1, wherein said host micro-organisms are contacted with said substrate in a growth medium comprising at least two antibiotic compounds as said growth regulator compounds for which resistance for each antibiotic compound is encoded in each of the operably linked coding regions, such that each operably linked coding region encodes resistance for one antibiotic compound.

4. A method according to claim 3, wherein the coding regions encode respective enzymes conferring antibiotic resistance.

5. A method according to claim 1, wherein at least one micro-organism host cell comprises one or more nucleic acid molecules encoding at least two primary modulator compounds each capable of catalysing at least one reaction of a multi-step chemical conversion reaction, and wherein at least one of said chemical conversion reactions produces said effector compound.

6. A method according to claim 1, wherein the micro-organism host cell comprises one or more nucleic acid molecules encoding at least two potential primary modulator compounds, wherein at least one primary modulator compound effects a chemical transformation of a substrate into said effector compound and at least one primary modulator compound transports said substrate into said microorganism.

7. A method according to any one of claims 1 to 5, further comprising:
- selecting the genetic coding sequence encoding at least one primary modulator compound from micro-organisms in said library which proliferate; and
- introducing the selected genetic coding sequence into a micro-organism host cell by transgenic methods thereby producing a production strain, wherein said production strain produces said primary modulator compound or compounds.

8. A library of micro-organism host cells suitable for use in a method of selecting among a population of potential primary modulator compounds a primary modulator compound, each said host cell comprising;
- at least one nucleic acid molecule encoding at least one potential primary modulator compound,
wherein said primary modulator compound
- effects a chemical transformation of a substrate into said effector compound and said substrate is provided to said microorganism host cells; or
- transports said effector compound into said micro-organism host cells; and
- at least one regulatable gene expression construct comprising a nucleic acid molecule encoding at least two or more regulation sequences, each said regulation sequence encoding an RNA molecule comprising a riboswitch operably linked to a respective coding region which encodes a respective secondary modulator compound for modulating the action of a respective growth regulator compound, wherein said at least two regulation sequences encode different secondary modulator compounds that act against different said at least two growth regulator compounds; and wherein each riboswitch regulates expression of its respective coding region in response to the presence of an effector compound.

9. A micro-organism host cell, wherein said host cell comprises:
at least one regulatable gene expression construct,
- wherein said regulatable gene expression construct comprises a nucleic acid molecule;
- wherein said nucleic acid molecule comprises two or more regulation sequences, each said regulation sequence encoding an RNA molecule comprising a riboswitch responsive to an effector compound, each said riboswitch being operably linked to a respective coding region;
- wherein each coding region encodes a respective modulator compound for modulating the action of a respective growth regulator compound;
- wherein said two or more regulation sequences encode different secondary modulator compounds that each act against a different growth regulator compound; and
wherein the riboswitch in each regulation sequence is selected to be responsive to the same effector compound to trigger expression of its respective modulator compound;
and at least one nucleic acid molecule encoding at least one primary modulator compound, wherein said primary modulator compound:
- effects a chemical transformation of a substrate into said effector compound; or
- transports a compound from the extracellular space to the intracellular space, wherein said transporter compound is said substrate or is said effector compound.

10. A micro-organism host cell as claimed in claim 9, wherein said host cell is a bacterial cell, a microbial cell, a plant cell, a yeast cell, or an animal cell.

## Patentansprüche

1. Verfahren für die In-vivo-Selektion einer genetischen Codierungssequenz, die mindestens eine primäre Modulatorverbindung codiert, die die Wirkung einer Effektorverbindung moduliert, umfassend Züchten einer Expressionsbibliothek von Wirtsmikroorganismen in Gegenwart von mindestens zwei Wachstumsregulierungsverbindungen, von welchen jede normalerweise imstande ist, eine Vermehrung der Mikroorganismen zu verhindern, wobei die Expressionsbibliothek eine Bibliothek von DNA-Fragmenten exprimiert, die potentiell die primäre Modulatorverbindung exprimieren, und Auswählen von Mikroorganismen in der Bibliothek, die sich vermehren;
wobei die primäre Modulatorverbindung
- eine chemische Transformation eines Substrats zu der Effektorverbindung bewirkt und das Substrat an die Mikroorganismen bereitgestellt wird; oder
- die Effektorverbindung in die Mikroorganismen transportiert und
wobei die Wirtsmikroorganismen ein regulierbares Genexpressionskonstrukt umfassen, wobei das regulierbare Genexpressionskonstrukt ein Nukleinsäuremolekül umfasst, wobei das Nukleinsäuremolekül mindestens zwei Regulierungssequenzen umfasst, wobei jede Regulierungssequenz für ein RNA-Molekül codiert, das einen Riboschalter umfasst, der funktionsfähig an eine entsprechende codierende Region gebunden ist, wobei jeder Riboschalter eine Expression seiner entsprechenden codierenden Region in Antwort auf das Vorhandensein der Effektorverbindung reguliert und jede codierende Region für eine entsprechende sekundäre Modulatorverbindung zum Modulieren der Wirkung einer entsprechenden Wachstumsregulierungsverbindung codiert, und wobei die mindestens zwei Regulierungssequenzen für verschiedene sekundäre Modulatorverbindungen codieren, die gegen verschiedene der mindestens zwei Wachstumsregulierungsverbindungen wirken;
wodurch ein Mikroorganismus in der Bibliothek, der eine gewünschte primäre Wachstumsregulierungsverbindung produziert, sich in Gegenwart der mindestens zwei Wachstumsregulierungsverbindungen vermehren kann.

2. Verfahren nach Anspruch 1, wobei die Expressionsbibliothek von Wirtsmikroorganismen eine Vielzahl von Wirtszellen umfasst, die eine Population von potentiellen primären Modulatorverbindungen produzieren, wobei die Vielzahl von Wirtszellen:
- eine Bibliothek von Zellen eines einzigen Zelltyps ist, wobei im Wesentlichen jede Wirtszelle ein geklontes Nukleinsäurefragment oder mutiertes natives Genom umfasst, das für mindestens eine potentielle primäre Modulatorverbindung codiert; oder
- Zellen verschiedener Zelltypen sind, wobei im Wesentlichen jede Wirtszelle ein natives Nukleinsäurefragment umfasst, das für mindestens eine potentielle primäre Modulatorverbindung codiert.

3. Verfahren nach Anspruch 1, wobei die Wirtsmikroorganismen mit dem Substrat in einem Wachstumsmedium in Kontakt gebracht werden, das mindestens zwei Antibiotikaverbindungen als die Wachstumsregulierungsverbindungen umfasst, für die Resistenz gegen jede Antibiotikaverbindung in jeder der funktionsfähig gebundenen, codierenden Regionen codiert ist, sodass jede funktionsfähig gebundene, codierende Region Resistenz für eine Antibiotikaverbindung codiert.

4. Verfahren nach Anspruch 3, wobei die codierenden Regionen entsprechende Enzyme codieren, die Antibiotikaresistenz verleihen.

5. Verfahren nach Anspruch 1, wobei mindestens eine Mikroorganismuswirtszelle ein oder mehrere Nukleinsäuremoleküle umfasst, die mindestens für zwei primäre Modulatorverbindungen codieren, von welchen jede imstande ist, mindestens eine Reaktion einer mehrstufigen chemischen Umsetzungsreaktion zu katalysieren und wobei mindestens eine der chemischen Umsetzungsreaktionen die Effektorverbindung produziert.

6. Verfahren nach Anspruch 1, wobei die Mikroorganismuswirtszelle ein oder mehrere Nukleinsäuremoleküle umfasst, die mindestens für zwei potentielle primäre Modulatorverbindungen codieren, wobei mindestens eine primäre Modulatorverbindung eine chemische Transformation eines Substrats in die Effektorverbindung bewirkt und mindestens eine primäre Modulatorverbindung das Substrat in den Mikroorganismus transportiert.

7. Verfahren nach einem der Ansprüche 1 bis 5, weiter umfassend:
- Selektieren der genetischen codierenden Sequenz, die für mindestens eine primäre Modulatorverbindung codiert, aus Mikroorganismen in der Bibliothek, die sich vermehren; und
- Einführen der ausgewählten genetischen Codierungssequenz in eine Mikroorganismuswirtszelle durch transgene Verfahren, wodurch ein Produktionsstamm produziert wird, wobei der Produktionsstamm die primäre Modulatorverbindung oder - verbindungen produziert.

8. Bibliothek von Mikroorganismuswirtszellen, die zur Verwendung in einem Verfahren zur Selektion einer primären Modulatorverbindung aus einer Population potentieller primärer Modulatorverbindungen geeignet ist, wobei jede Wirtszelle umfasst:
- mindestens ein Nukleinsäuremolekül, das für mindestens eine potentielle primäre Modulatorverbindung codiert;
wobei die primäre Modulatorverbindung
- eine chemische Transformation eines Substrats zu der Effektorverbindung bewirkt und das Substrat an die Mikroorganismuswirtszellen bereitgestellt wird; oder
- die Effektorverbindung in die Mikroorganismen transportiert und
- mindestens ein regulierbares Genexpressionskonstrukt, das ein Nukleinsäuremolekül umfasst, das für mindestens zwei oder mehr Regulierungssequenzen codiert, wobei jede der Regulierungssequenzen für ein RNA-Molekül codiert, das einen Riboschalter umfasst, der funktionsfähig an eine entsprechende codierende Region gebunden ist, die für eine entsprechende sekundäre Modulatorverbindung zum Modulieren der Wirkung einer entsprechenden Wachstumsregulierungsverbindung codiert, wobei die mindestens zwei Regulierungssequenzen für verschiedene sekundäre Modulatorverbindungen codieren, die gegen verschiedene der mindestens zwei Wachstumsregulierungsverbindungen wirken; und wobei der Riboschalter in Antwort auf das Vorhandensein einer Effektorverbindung eine Expression seiner entsprechenden codierenden Region reguliert.

9. Mikroorganismuswirtszelle, wobei die Wirtszelle umfasst:
mindestens ein regulierbares Genexpressionskonstrukt,
- wobei das regulierbare Genexpressionskonstrukt ein Nukleinsäuremolekül umfasst;
- wobei das Nukleinsäuremolekül zwei oder mehr Regulierungssequenzen umfasst, wobei jede Regulierungssequenz für ein RNA-Molekül codiert, das einen Riboschalter umfasst, der auf eine Effektoverbindung anspricht, wobei jeder Riboschalter funktionsfähig an eine entsprechende codierende Region gebunden ist;
- wobei jede codierende Region für eine entsprechende Modulatorverbindung zum Modulieren der Wirkung einer entsprechenden Wachstumsregulierungsverbindung codiert;
- wobei die zwei oder mehr Regulierungssequenzen für verschiedene sekundäre Modulatorverbindungen codieren, die jeweils gegen eine andere Wachstumsregulierungsverbindung wirken; und
- wobei der Riboschalter in jeder Regulierungssequenz selektiert ist, auf dieselbe Effektorverbindung anzusprechen, um eine Expression seiner entsprechenden Modulatorverbindung auszulösen;
und mindestens ein Nukleinsäuremolekül, das für mindestens eine primäre Modulatorverbindung codiert, wobei die primäre Modulatorverbindung:
- eine chemische Transformation eines Substrats zu der Effektorverbindung bewirkt; oder
- eine Verbindung aus dem extrazellulären Raum zum intrazellulären Raum transportiert, wobei die Transporterverbindung das Substrat oder die Effektorverbindung ist.

10. Mikroorganismuswirtszelle nach Anspruch 9, wobei die Wirtszelle eine bakterielle Zelle, eine mikrobielle Zelle, eine Pflanzenzelle, eine Hefezelle oder eine Tierzelle ist.

## Revendications

1. Procédé pour la sélection in vivo d'une séquence codante génétique codant pour au moins un composé modulateur primaire modulant l'action d'un composé effecteur, comprenant la croissance d'une banque d'expression de micro-organismes hôtes en présence d'au moins deux composés régulateurs de croissance capables chacun normalement d'empêcher la prolifération desdits micro-organismes, laquelle banque d'expression exprime une banque de fragments d'ADN exprimant potentiellement ledit composé modulateur primaire, et la sélection de micro-organismes dans ladite banque qui prolifèrent ;
dans lequel ledit composé modulateur primaire
- effectue une transformation chimique d'un substrat en ledit composé effecteur et ledit substrat est fourni auxdits micro-organismes ; ou
- transporte ledit composé effecteur dans lesdits micro-organismes, et
dans lequel lesdits micro-organismes hôtes comprennent une construction d'expression génique pouvant être régulée, ladite construction d'expression génique pouvant être régulée comprenant une molécule d'acide nucléique, ladite molécule d'acide nucléique comprenant au moins deux séquences de régulation, chaque séquence de régulation codant pour une molécule d'ARN comprenant un riborégulateur lié de manière fonctionnelle à une région codante respective, dans lequel chaque riborégulateur régule l'expression de sa région codante respective en réponse à la présence dudit composé effecteur, et chaque région codante code pour un composé modulateur secondaire respectif pour moduler l'action d'un desdits composés régulateurs de croissance respectifs, et dans lequel lesdites au moins deux séquences de régulation codent pour des composés modulateurs secondaires différents qui agissent contre lesdits au moins deux composés régulateurs de croissance différents ;
moyennant quoi un micro-organisme dans ladite banque qui produit un composé modulateur primaire souhaité est autorisé à proliférer en présence desdits au moins deux composés régulateurs de croissance.

2. Procédé selon la revendication 1, dans lequel la banque d'expression de micro-organismes hôtes comprend une multitude de cellules hôtes qui produisent une population de composés modulateurs primaires potentiels, dans lequel ladite multitude de cellules hôtes :
- est une banque de cellules d'un type de cellule unique, dans lequel essentiellement chaque cellule hôte comprend un fragment d'acide nucléique cloné ou un génome natif muté codant pour au moins un composé modulateur primaire potentiel ; ou
- sont des cellules de différents types de cellules, dans lequel essentiellement chaque cellule hôte comprend un fragment d'acide nucléique natif codant pour au moins un composé modulateur primaire potentiel.

3. Procédé selon la revendication 1, dans lequel lesdits micro-organismes hôtes sont mis en contact avec ledit substrat dans un milieu de croissance comprenant au moins deux composés antibiotiques en tant que lesdits composés régulateurs de croissance pour lesquels une résistance pour chaque composé antibiotique est codée dans chacune des régions codantes liées de manière fonctionnelle, de sorte que chaque région codante liée de manière fonctionnelle code pour une résistance pour un composé antibiotique.

4. Procédé selon la revendication 3, dans lequel les régions codantes codent pour des enzymes respectives conférant une résistance aux antibiotiques.

5. Procédé selon la revendication 1, dans lequel au moins une cellule hôte de micro-organisme comprend une ou plusieurs molécules d'acide nucléique codant pour au moins deux composés modulateurs primaires capables chacun de catalyser au moins une réaction d'une réaction de conversion chimique à plusieurs étapes, et dans lequel au moins une desdites réactions de conversion chimique produit ledit composé effecteur.

6. Procédé selon la revendication 1, dans lequel la cellule hôte de micro-organisme comprend une ou plusieurs molécules d'acide nucléique codant pour au moins deux composés modulateurs primaires potentiels, dans lequel au moins un composé modulateur primaire effectue une transformation chimique d'un substrat en ledit composé effecteur et au moins un composé modulateur primaire transporte ledit substrat dans ledit micro-organisme.

7. Procédé selon l'une quelconque des revendications 1 à 5, comprenant en outre :
- la sélection de la séquence codante génétique codant pour au moins un composé modulateur primaire issu de micro-organismes dans ladite banque qui prolifèrent ; et
- l'introduction de la séquence codante génétique sélectionnée dans une cellule hôte de micro-organisme par des procédés transgéniques en produisant ainsi une souche de production, dans lequel ladite souche de production produit ledit composé ou lesdits composés modulateurs primaires.

8. Banque de cellules hôtes de micro-organisme adéquate pour une utilisation dans un procédé de sélection parmi une population de composés modulateurs primaires potentiels d'un composé modulateur primaire, chacune desdites cellules hôtes comprenant ;
- au moins une molécule d'acide nucléique codant pour au moins un composé modulateur primaire potentiel,
dans laquelle ledit composé modulateur primaire
- effectue une transformation chimique d'un substrat en ledit composé effecteur et ledit substrat est fourni auxdites cellules hôtes de micro-organisme ; ou
- transporte ledit composé effecteur dans lesdites cellules hôtes de micro-organisme ; et
- au moins une construction d'expression génique pouvant être régulée comprenant une molécule d'acide nucléique codant pour au moins deux séquences de régulation ou plus, chacune desdites séquences de régulation codant pour une molécule d'ARN comprenant un riborégulateur lié de manière fonctionnelle à une région codante respective qui code pour un composé modulateur secondaire respectif pour moduler l'action d'un composé régulateur de croissance respectif, dans laquelle lesdites au moins deux séquences de régulation codent pour différents composés modulateurs secondaires qui agissent contre lesdits au moins deux composés régulateurs de croissance différents ; et dans laquelle chaque riborégulateur régule l'expression de sa région codante respective en réponse à la présence d'un composé effecteur.

9. Cellule hôte de micro-organisme, dans laquelle ladite cellule hôte comprend :
au moins une construction d'expression génique pouvant être régulée,
- dans laquelle ladite construction d'expression génique pouvant être régulée comprend une molécule d'acide nucléique ;
- dans laquelle ladite molécule d'acide nucléique comprend deux séquences de régulation ou plus, chacune desdites séquences de régulation codant pour une molécule d'ARN comprenant un riborégulateur réagissant à un composé effecteur, chacun desdits riborégulateurs étant lié de manière fonctionnelle à une région codante respective ;
- dans laquelle chaque région codante code pour un composé modulateur respectif pour moduler l'action d'un composé régulateur de croissance respectif ;
- dans laquelle lesdites deux séquences de régulation ou plus codent pour différents composés modulateurs secondaires qui agissent chacun contre un composé régulateur de croissance différent ; et
dans laquelle le riborégulateur dans chaque séquence de régulation est sélectionné pour être réactif au même composé effecteur pour déclencher l'expression de son composé modulateur respectif ;
et au moins une molécule d'acide nucléique codant pour au moins un composé modulateur primaire, dans laquelle ledit composé modulateur primaire :
- effectue une transformation chimique d'un substrat en ledit composé effecteur ; ou
- transporte un composé de l'espace extracellulaire vers ledit espace intracellulaire, dans laquelle ledit composé transporteur est ledit substrat ou est ledit composé effecteur.

10. Cellule hôte de micro-organisme selon la revendication 9, dans laquelle ladite cellule hôte est une cellule bactérienne, une cellule microbienne, une cellule végétale, une cellule de levure ou une cellule animale.
